(19) Europäisches Patentamt / European Patent Office / Office européen des brevets

(11) **EP 4 122 490 A1**

(12) **EUROPEAN PATENT APPLICATION**
published in accordance with Art. 153(4) EPC

(43) Date of publication:
**25.01.2023 Bulletin 2023/04**

(21) Application number: **21774984.5**

(22) Date of filing: **24.03.2021**

(51) International Patent Classification (IPC):
*A61K 38/48* (2006.01)     *A61P 25/16* (2006.01)
*C07K 14/435* (2006.01)

(52) Cooperative Patent Classification (CPC):
**A61K 38/48; A61P 25/16; C07K 14/435**

(86) International application number:
**PCT/CN2021/082715**

(87) International publication number:
**WO 2021/190561 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.03.2020 CN 202010212922**

(71) Applicant: **Talengen International Limited
Hong Kong 999077 (HK)**

(72) Inventor: **LI, Jinan
Beijing 100089 (CN)**

(74) Representative: **karo IP
karo IP Patentanwälte
Kahlhöfer Rößler Kreuels PartG mbB
Postfach 32 01 02
40416 Düsseldorf (DE)**

Remarks:
The complete document including Reference
Table(s) and the Sequence Listing(s) can be
downloaded from the EPO website

(54) **METHOD AND DRUG FOR TREATING PARKINSON'S DISEASE**

(57)     Provided is a method for preventing and treating Parkinson's disease, including administering to a subject a therapeutically effective amount of a component of a plasminogen activation pathway. The present invention relates to a drug, a pharmaceutical composition, a product and a kit which include the component of the plasminogen activation pathway for treating the described condition.

EP 4 122 490 A1

## Description

FIELD OF THE INVENTION

The present invention relates to a method for treating Parkinson's disease, including: administering an effective amount of a component of a plasminogen activation pathway or a related compound thereof, such as plasminogen, to a subject in order to improve clinical symptoms and signs.

BACKGROUND OF THE INVENTION

Parkinson's disease (PD) is a common neurodegenerative disease, which is more common in the elderly, with an average age of onset around 60 years old. Young people with onset of Parkinson's disease under the age of 40 are relatively rare. Most patients with Parkinson's disease are sporadic cases, in which less than 10% of patients have a family history. The most important pathological change in Parkinson's disease is the degeneration and death of dopamine (DA)-rgic neurons in the substantia nigra, resulting in a significant decrease in striatal DA content and causing disease. The exact cause of this pathological change is still unclear. Genetic factors, environmental factors, aging, oxidative stress and the like may all be involved in the degeneration and death of PD dopaminergic neurons.

The prominent pathological changes of Parkinson's disease are the degeneration and death of DA neurons in the midbrain substantia nigra, the marked reduction of striatal DA content, and the appearance of eosinophilic inclusion bodies in the cytoplasm of the remaining neurons in the substantia nigra, namely Lewy body. In addition to a dopaminergic system, non-dopaminergic systems are also significantly impaired in patients with Parkinson's disease, such as cholinergic neurons in the basal nucleus of Meynert, noradrenergic neurons in the locus coeruleus, 5-hydroxytryptaminergic neurons in the raphe nucleus of the brainstem, and neurons in the cerebral cortex, brainstem, spinal cord and peripheral autonomic nervous system. A significant decrease in striatal dopamine level is closely related to the appearance of motor symptoms in Parkinson's disease. The significant decrease in the concentration of dopamine in a mesolimbic system and a midbrain-cortical system is closely related to the decline of intelligence and affective disorders in patients with Parkinson's disease.

The onset of Parkinson's disease is insidious and the progress is slow. The first symptom is usually tremor or clumsiness of one limb, which can then involve the opposite limb. The main clinical manifestations include resting tremor, bradykinesia, myotonia, and postural Instability and gait disorder. In recent years, it has been increasingly noticed that non-motor symptoms such as depression, constipation and sleep disturbance are also common complaints of patients with Parkinson's disease, and their impacts on the quality of life of patients even exceed that of motor symptoms.

Drug therapy is the main treatment method for Parkinson's disease, which can improve the symptoms to a certain extent, but cannot prevent the progress of the disease. Therefore, other treatment methods and drugs need to be found.

SUMMARY OF THE INVENTION

In the present invention, it is found through researches that plasminogen can promote the recovery of a memory function, improve the cognitive ability, promote the expression of DTA in the substantia nigra, promote the recovery of the Nissl body of striatum, promote the expression of GLP-1R in the substantia nigra, increase the number of TH-positive cells in the substantia nigra, promote the myelin sheath repair of striatum, promote the degradation of $\alpha$-synuclein in brain tissues, promote the NF expression of striatum, promote the axonal damage repair, reduce the GFAP expression of striatum, reduce the neuron damage of striatum, promote the cleavage of Pro-BDNF in brain tissues into BDNF, and relieve the depression or anxiety symptoms, thereby having the potential to be developed into a drug for the treatment of Parkinson's disease.

Specifically, the present invention relates to the following items.

1. In one aspect, the present application relates to a method for preventing and treating Parkinson's disease, including: administering a therapeutically effective amount of one or more compounds to a subject with Parkinson's disease, the one or more compounds being selected from the group consisting of: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog and an antagonist of a fibrinolysis inhibitor.

In one aspect, the present application relates to use of one or more compounds in the preparation of a drug for treating Parkinson's disease, the one or more compounds being selected from the group consisting of: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog and an antagonist of a fibrinolysis inhibitor.

In one aspect, the present application relates to a drug or a pharmaceutical composition that is used for treating Parkinson's disease and contains one or more compounds, the one or more compounds being selected from the group consisting of: a component of a plasminogen activation pathway, a compound capable of directly activating plasminogen or indirectly activating plasminogen by activating an upstream component of a plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog and an antagonist of a fibrinolysis inhibitor.

2. According to the method, the use, the drug or the pharmaceutical composition according to Item 1, the component of the plasminogen activation pathway is selected from the group consisting of plasminogen, recombinant human plasmin, Lys-plasminogen, Glu-plasminogen, plasmin, plasminogen and plasmin variants and analogs containing one or more kringle domains and protease domains of plasminogen and plasmin, miniplasminogen, mini-plasmin, micro-plasminogen, microplasmin, delta-plasminogen, delta-plasmin, a plasminogen activator, tPA and uPA.

3. According to the method, the use, the drug or the pharmaceutical composition according to Item 1, the antagonist of the fibrinolysis inhibitor is an inhibitor of PAI-1, a complement C1 inhibitor, $\alpha 2$ antiplasmin or an $\alpha 2$ macroglobulin, such as an antibody.

4. According to the method, the use, the drug or the pharmaceutical composition according to any one of Items 1 to 3, the compound has one or more of the following effects on a subject with Parkinson's disease: promoting the recovery of a memory function, improving the cognitive ability, promoting the expression of DTA in the substantia nigra, promoting the recovery of the Nissl body of striatum, promoting the expression of GLP-1R in the substantia nigra, increasing the number of TH-positive cells in the substantia nigra, promoting the myelin sheath repair of striatum, promoting the degradation of $\alpha$-synuclein in brain tissues, promoting the NF expression of striatum, promoting the axonal damage repair, reducing the GFAP expression of striatum, reducing the neuron damage of striatum, promoting the cleavage of Pro-BDNF in brain tissues into BDNF, and relieving the depression or anxiety symptoms.

5. According to the method, the use, the drug or the pharmaceutical composition according to any one of Items 1 to 4, wherein the compound is plasminogen.

6. According to the method, the use, the drug or the pharmaceutical composition according to any one of Items 1 to 5, the plasminogen is human full-length plasminogen or a conservatively substituted variant thereof.

7. According to the method, the use, the drug or the pharmaceutical composition according to any one of Items 1 to 5, the plasminogen has at least 75%, 80%, 85%, 90%, 95%, 96%, 97%, 98% or 99% sequence identity to sequence 2 and still has a lysine binding activity or proteolytic activity of the plasminogen.

8. According to the method, the use, the drug or the pharmaceutical composition according to any one of Items 1 to 5, the plasminogen contains a protein that has at least 80%, 90%, 95%, 96%, 97%, 98% or 99% amino acid sequence identity to sequence 14 and still has a proteolytic activity of the plasminogen.

9. According to the method, the use, the drug or the pharmaceutical composition according to any one of Items 1 to 5, the plasminogen is selected from the group consisting of Glu-plasminogen, Lys-plasminogen, small plasminogen, microplasminogen, delta-plasminogen or their variants that retain a proteolytic activity of the plasminogen.

10. According to the method, the use, the drug or the pharmaceutical composition according to any one of Items 1 to 5, the plasminogen contains an amino acid sequence shown in sequence 2, 6, 8, 10, 12 or a conservatively substituted variant containing an amino acid sequence shown in sequence 2, 6, 8, 10, 12.

11. According to the method, the use, the drug or the pharmaceutical composition according to any one of Items 1 to 10, the compound is used in combination with one or more other therapeutic methods or drugs.

12. According to the method, the use, the drug or the pharmaceutical composition according to Item 11, the other therapeutic methods include a cell therapy (including a stem cell therapy) and a physical therapy.

13. According to the method, the use, the drug or the pharmaceutical composition according to Item 11, the other drugs are other drugs for the treatment of Parkinson's disease.

14. According to the method, the use, the drug or the pharmaceutical composition according to any one of Items 1 to 13, the compound is administered by means of nasal inhalation, aerosol inhalation, nasal drops, eye drops, ear drops, an intravenous method, an intraperitoneal method, a subcutaneous method, an intracranial method, an intrathecal method, an intraarterial method (e.g., via the carotid artery) or an intramuscular method. In any of the above embodiments of the present invention, the plasminogen has at least 75%, 80%, 85%, 90%, 95%, 96%, 97%, 98% or 99% sequence identity to sequence 2, 6, 8, 10 or 12, and still has an activity, e.g., a lysine binding activity or proteolytic activity, of plasminogen. In some embodiments, the plasminogen is a protein that is obtained by adding, deleting and/or substituting 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid on the basis of sequence 2, 6, 8, 10 or 12, and still has the activity, such as the lysine binding activity and the proteolytic activity, of plasminogen.

In some embodiments, the plasminogen is a protein that contains a plasminogen active fragment, and still has the activity, e.g., the lysine binding activity or the proteolytic activity, of the plasminogen. In some embodiments, the plasminogen is selected from the group consisting of Glu-plasminogen, Lys-plasminogen, small plasminogen, microplasminogen, delta-plasminogen or their variants that retain the activity, e.g., the proteolytic activity, of the plasminogen. In some embodiments, the plasminogen is natural or synthetic human plasminogen, or a variant or fragment thereof that still has the activity, e.g., the lysine binding activity or the proteolytic activity, of the plasminogen. In some embodiments, the plasminogen is a human plasminogen ortholog from a primate or rodent, or a variant or fragment thereof that still has the activity, e.g., the lysine binding activity or the proteolytic activity, of the plasminogen. In some embodiments, the amino acid of the plasminogen is shown in sequence 2, 6, 8, 10 or 12. In some embodiments, the plasminogen is human natural plasminogen.

In some embodiments, the subject is a human. In some embodiments, the subject is lack of or deficient in plasminogen. In some embodiments, the lack or deficiency is congenital, secondary and/or local.

In some embodiments, the pharmaceutical composition includes a pharmaceutically acceptable carrier and the plasminogen for use in the above-mentioned method. In some embodiments, the kit may be a preventive or therapeutic kit including: (i) the plasminogen for use in the above-mentioned method, and (ii) a means for delivering the plasminogen to the subject. In some embodiments, the means is a syringe or a vial. In some embodiments, the kit further includes a label or an instruction for use indicating the administration of the plasminogen to the subject to implement any one of the above-mentioned methods.

In some embodiments, the product includes: a container including a label; and (i) the plasminogen for use in the above-mentioned methods or a pharmaceutical composition including the plasminogen, wherein the label indicates the administration of the plasminogen or the composition to the subject to implement any one of the above-mentioned methods.

In some embodiments, the kit or the product further includes one or more additional means or containers containing other drugs.

In some embodiments of the above-mentioned methods, the plasminogen is administered by systemic or topical route, preferably by the following routes: intravenous, intramuscular, and subcutaneous administration of plasminogen for treatment. In some embodiments of the above-mentioned methods, the plasminogen is administered in combination with a suitable polypeptide carrier or stabilizer. In some embodiments of the above-mentioned methods, the plasminogen is administered at a dosage of 0.0001-2000 mg/kg, 0.001-800 mg/kg, 0.01-600 mg/kg, 0.1-400 mg/kg, 1-200 mg/kg, 1-100 mg/kg or 10-100 mg/kg (by per kg of body weight) or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$ or 10-100 mg/cm$^2$ (by per square centimeter of body surface area) daily, preferably the dosage is repeated at least once, preferably the dosage is administered at least daily.

The present invention explicitly encompasses all the combinations of technical features belonging to the embodiments of the present invention, and these combined technical solutions have been explicitly disclosed in the present application, as if the above-mentioned technical solutions are individually and explicitly disclosed. In addition, the present invention also explicitly encompasses all the combinations between various embodiments and elements thereof, and the combined technical solutions are explicitly disclosed herein.

## BRIEF DESCRIPTION OF THE DRAWINGS

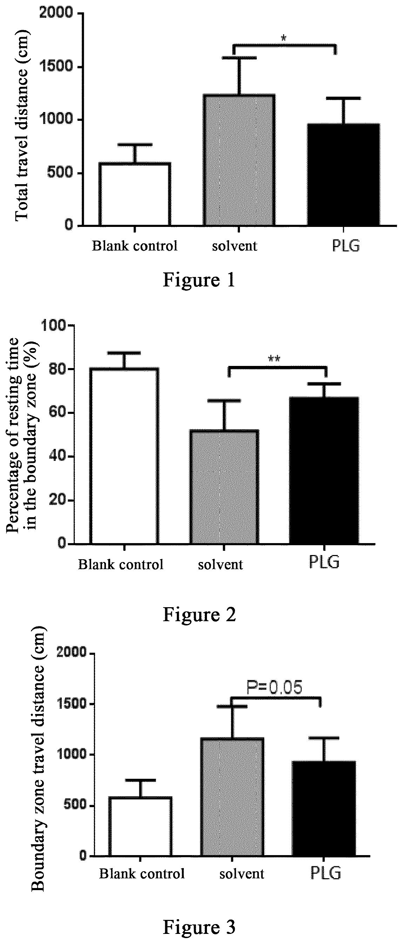

FIG. 1 shows statistical results of a total travel distance of Parkinson's model mice in an open field test after 14 days of administration of plasminogen. The results show that each mouse in a blank control group travels for a certain distance during the test; a total travel distance of the mouse in a plasminogen administration group is significantly shorter than that in a solvent control group, and a statistical difference is significant (* indicates P<0.05); and a total travel distance of each mouse in a plasminogen administration group is close to that in the blank control group. This indicates that the plasminogen can enhance the phobotaxis of Parkinson's model mice and relieve their anxiety.

FIG. 2 shows statistical results of a resting time rate in a boundary zone of Parkinson's model mice in an open field test after 14 days of administration of plasminogen. The results show that each mouse in the blank control group has a certain resting time rate in the boundary zone; the resting time rate in the boundary zone of each mouse in the plasminogen administration group is significantly greater than that in the solvent control group, and the statistical difference is extremely significant (** indicates P<0.01); and the resting time rate in the boundary zone of each mouse in the plasminogen administration group is close to that in the blank control group. This indicates that the plasminogen can enhance the phobotaxis of Parkinson's model mice and relieve their anxiety.

FIG. 3 shows statistical results of a travel distance in a boundary zone of Parkinson's model mice in an open field test after 14 days of administration of plasminogen. The results show that each mouse in the blank control group has a certain travel distance in the boundary zone; the travel distance in the boundary zone of each mouse in the plasminogen administration group is significantly shorter than that in the solvent control group, and the statistical difference is close to significant (P=0.05); and the travel distance in the boundary zone of each mouse in the plasminogen administration group is close to that in the blank control group. This indicates that the plasminogen can enhance the phobotaxis of Parkinson's model mice and relieve their anxiety.

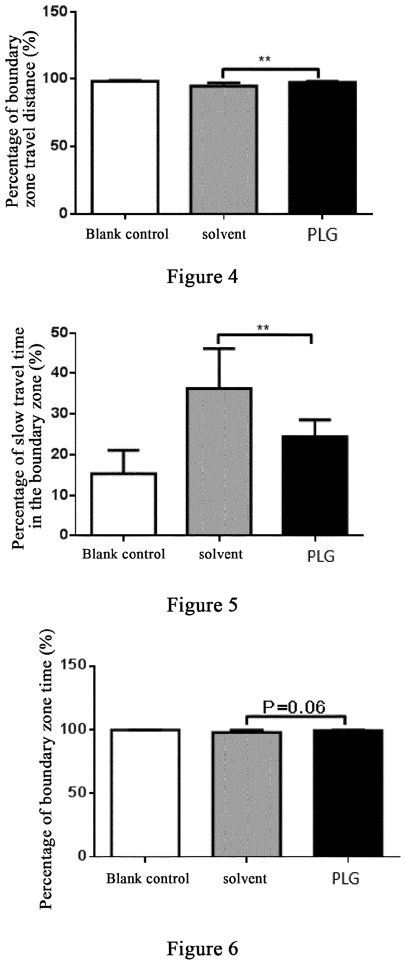

FIG. 4 shows statistical results of a travel distance percentage in a boundary zone of Parkinson's model mice in an open field test after 14 days of administration of plasminogen. The results show that each mouse in the blank control group has a certain percentage of a travel distance in the boundary zone; the percentage of the travel distance in the boundary zone of each mouse in the plasminogen administration group is significantly greater than that in the solvent control group, and the statistical difference is extremely significant (** indicates P<0.01); and the percentage of the travel distance in the boundary zone of each mouse in the plasminogen administration group is close to that in the blank control group. This indicates that the plasminogen can enhance the phobotaxis of Parkinson's model mice and relieve their anxiety.

FIG. 5 shows calculation results of a percentage of slow travel time in a boundary zone of Parkinson's model mice in an open field test after 14 days of administration of plasminogen. The results show that each mouse in the blank control group has a certain percentage of slow travel time in the boundary zone; the percentage of the slow travel time in the boundary zone of each mouse in the plasminogen administration group is significantly less than that in the solvent control group, and the statistical difference is extremely significant (** indicates P<0.01); and the percentage of the slow travel time in the boundary zone of each mouse in the plasminogen administration group is close to that in the blank control group. This indicates that the plasminogen can enhance the phobotaxis of Parkinson's model mice and relieve their anxiety.

FIG. 6 shows statistical results of a time percentage in a boundary zone of Parkinson's model mice in an open field test after 14 days of administration of plasminogen. The results show that each mouse in the blank control group has a certain time percentage in the boundary zone; the time percentage in the boundary zone of each mouse in the plasminogen administration group is significantly greater than that in the solvent control group, and the statistical difference is close to significant (P=0.06); and the time percentage in the boundary zone of each mouse in the plasminogen administration group is close to that in the blank control group. This indicates that the plasminogen can enhance the phobotaxis behavior level of Parkinson's model mice and relieve their anxiety.

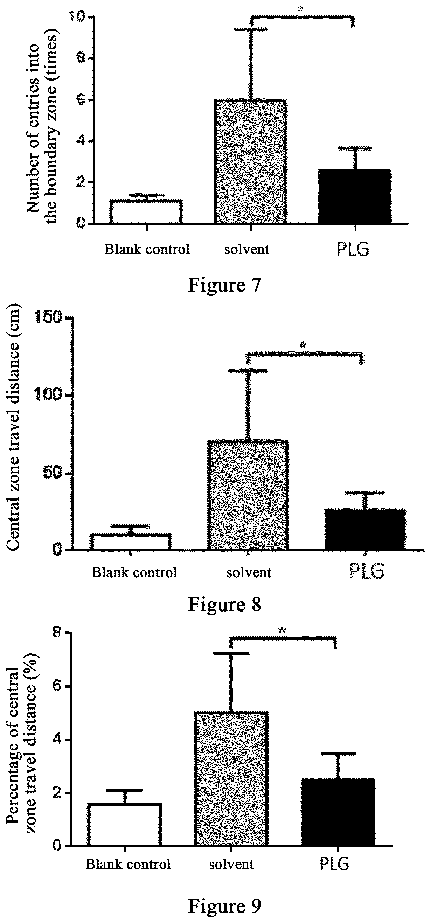

FIG. 7 shows statistical results of the number of entries of the Parkinson's model mice into a boundary zone in an open field test after 14 days of administration of plasminogen. The results show that each mouse in the blank control group has a certain number of entries into the boundary zone; the number of entries of each mouse in the plasminogen administration group into the boundary zone is significantly less than that in the solvent group, and the statistical difference is significant (* indicates P<0.05); and the number of entries of each mouse in the plasminogen administration group into the boundary zone is close to that in the blank control group. This indicates that the plasminogen can enhance the phobotaxis of Parkinson's model mice and relieve their anxiety.

FIG. 8 shows statistical results of a travel distance in a central zone of Parkinson's model mice in an open field test after 14 days of administration of plasminogen. The results show that each mouse in the blank control group has a certain travel distance in the central zone; the travel distance in the central zone of each mouse in the plasminogen administration group is significantly shorter than that in the solvent control group, and the statistical difference is significant (* indicates P<0.05); and the travel distance in the central zone of each mouse in the plasminogen administration group is close to that in the blank control group. This indicates that the plasminogen can enhance the phobotaxis of Parkinson's model mice and relieve their anxiety.

FIG. 9 shows statistical results of a percentage of a travel distance in a central zone of Parkinson's model mice in an open field test after 14 days of administration of plasminogen. The results show that each mouse in the blank control group has a certain percentage of a travel distance in the central zone; the percentage of the travel distance in the central zone of each mouse in the plasminogen administration group is significantly less than that in the solvent control group, and the statistical difference is significant (* indicates P<0.05); and the percentage of the travel distance in the central zone of each mouse in the plasminogen administration group is close to that in the blank control group. This indicates that the plasminogen can enhance the phobotaxis of Parkinson's model mice and relieve their anxiety.

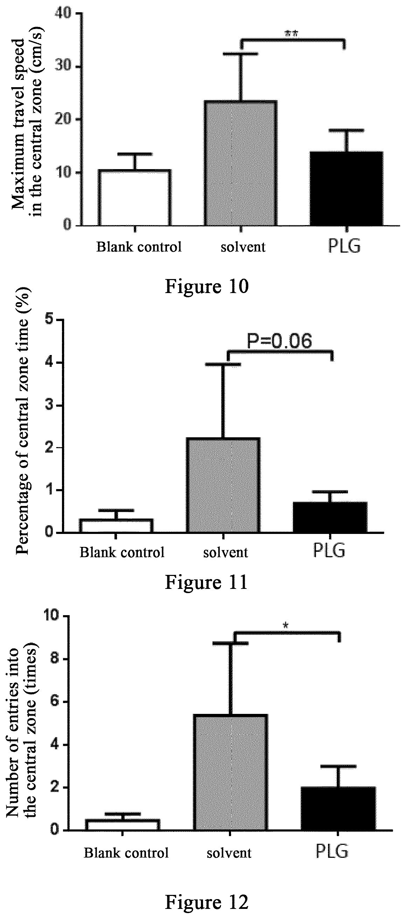

FIG. 10 shows statistical results of a maximum travel speed in a central zone of Parkinson's model mice in an open field test after 14 days of administration of plasminogen. The results show that each mouse in the blank control group has a certain maximum travel speed in the central zone; the maximum travel speed in the central zone of each mouse in the plasminogen administration group is significantly less than that in the solvent control group, and the statistical difference is extremely significant (** indicates P<0.01); and the maximum travel speed in the central zone of each mouse in the plasminogen administration group is close to that in the blank control group. This indicates that the plasminogen can enhance the phobotaxis of Parkinson's model mice and relieve their anxiety.

FIG. 11 shows statistical results of a time percentage in a center zone of Parkinson's model mice in an open field test after 14 days of administration of plasminogen. The results show that each mouse in the blank control group has a certain time percentage in the central zone; the time percentage in the central zone of each mouse in the plasminogen administration group is significantly less than that in the solvent control group, and the statistical difference is close to significant (P=0.06); and the time percentage in the central zone of each mouse in the plasminogen administration group is close to that in the blank control group. This indicates that the plasminogen can enhance the phobotaxis of Parkinson's model mice and relieve their anxiety.

FIG. 12 shows statistical results of the number of entries of the Parkinson's model mice into a central zone in an open field test after 14 days of administration of plasminogen. The results show that each mouse in the blank control group has a certain number of entries into the central zone; the number of entries of each mouse in the plasminogen administration group into the central zone is significantly less than that in the solvent control group, and the statistical difference is significant (* indicates P<0.05); and the number of entries of each mouse in the plasminogen administration group into the central zone is close to that in the blank control group. This indicates that the plasminogen can enhance the phobotaxis of Parkinson's model mice and relieve their anxiety.

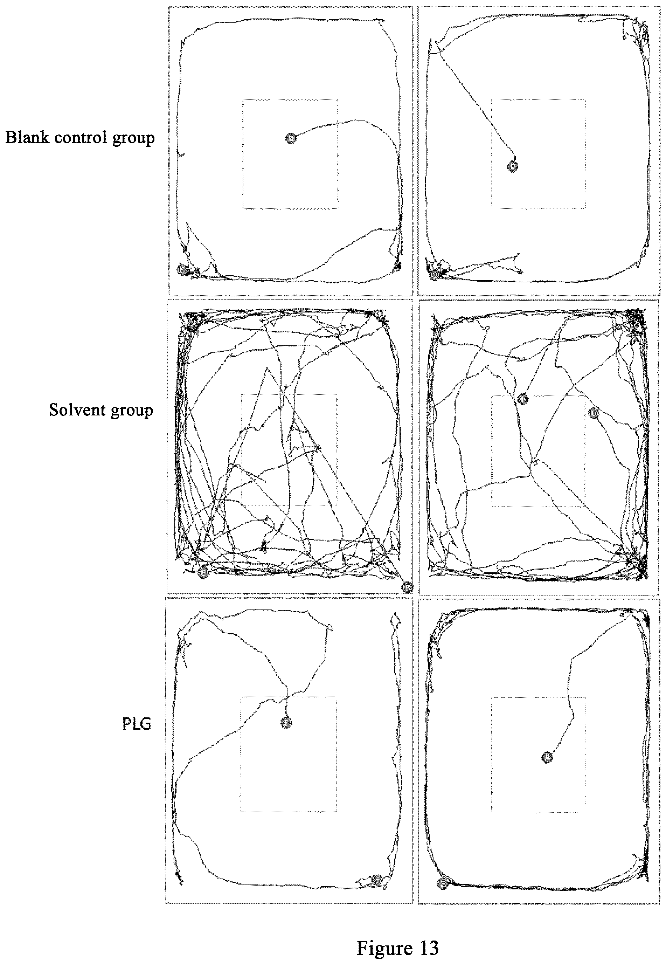

FIG. 13 shows a representative picture of travel trails of Parkinson's model mice in an open field test after 14 days of administration of plasminogen. The results show that each mouse in the blank control group has almost no activity in the central zone; and a trend of significantly reduced activities in the central zone reflected by each mouse in the plasminogen administration group is close to that in the blank control group compared with that in the solvent control group. This indicates that the plasminogen can enhance the phobotaxis of Parkinson's model mice and relieve their anxiety.

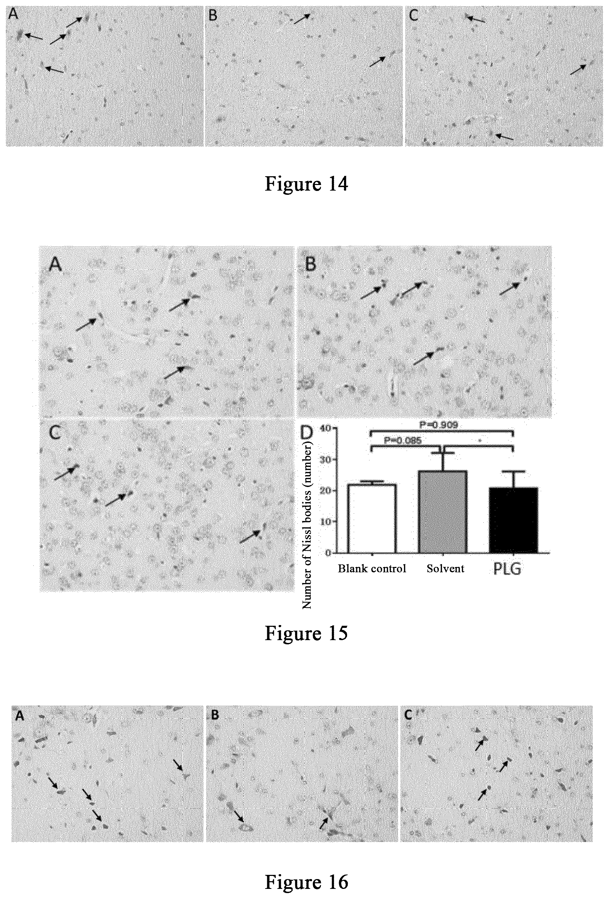

FIGS. 14A-C show DTA immunohistochemical results of substantia nigra in Parkinson's model mice in an open field test after 14 days of administration of plasminogen. A shows the blank control group, B shows the solvent control group, and C shows the plasminogen administration group. The results show that dopamine neurons in the substantia nigra of each mouse in the blank control group expresses a certain amount of DTA (marked by arrows), and the expression quantity of DTA in the substantia nigra of each mouse in the solvent group is lower than that in the blank control group, while the expression quantity of DTA in the substantia nigra of each mouse in the plasminogen administration group is higher than that in the solvent group, and is close to that in the blank control group. The results indicate that the plasminogen can promote the expression of DTA in the substantia nigra of Parkinson's model mice.

FIGS. 15A-D show tar violet staining results of the striatum of Parkinson's model mice in an open field test after 14 days of administration of plasminogen. A shows the blank control group, B shows the solvent control group, C shows the plasminogen administration group, and D shows analysis results of the number of Nissl bodies of the striatum. The results show that striatal neurons of each mouse in the blank control group have a certain number of Nissl bodies (marked by arrows); the number of Nissl bodies in striatal neurons of each mouse in the solvent control group is significantly more than that in the blank control group, and the statistical difference is close to significant (P=0.085); the number of Nissl bodies in striatal neurons of each mouse in the plasminogen administration group has no significant difference from that in the blank control group, but is significantly lower than that in the solvent control group, and the statistical difference is significant (* indicates P<0.05). The results indicate that the plasminogen can affect the number of Niss1 bodies of the striatum in Parkinson's model mice.

FIGS. 16A-C show tar violet staining results of the striatum of Parkinson's model mice after 14 days of administration of plasminogen. A shows the blank control group, B shows the solvent control group, and C shows the plasminogen administration group. The results show that each mouse in the blank control group has a certain amount of Nissl bodies (marked by arrows) in the substantia nigra; the number of Nissl bodies in the substantia nigra of each mouse in the solvent control group is less than that in the blank control group; and the number of Nissl bodies in the substantia nigra of each mouse in the plasminogen administration group is higher than that in the solvent group. The results indicate that the plasminogen can promote the recovery of Nissl bodies in the substantia nigra of Parkinson's model mice.

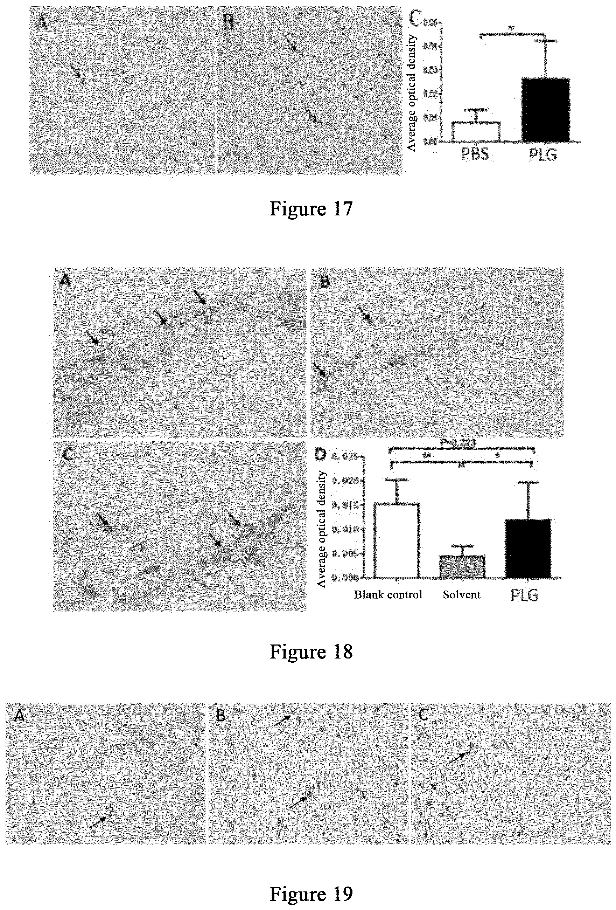

FIGS. 17A-C show GLP-1R immunohistochemical results of substantia nigra in Parkinson's model mice after 14 days of administration of plasminogen. A shows a solvent PBS group, B shows the plasminogen administration group, and C shows a quantitative analysis result of the average optical density. The results show that the expression quantity of GLP-1R in the substantia nigra of each mouse in the plasminogen administration group (marked by arrows) is significantly higher than that in the solvent PBS control group, and the statistical difference is significant (* indicates P<0.05). The results indicate that the plasminogen can promote the expression of GLP-1R in the substantia nigra of Parkinson's model mice.

FIGS. 18A-D show TH immunohistochemical results of substantia nigra in Parkinson's model mice after 14 days of administration of plasminogen. A shows the blank control group, B shows the solvent PBS group, C shows the plasminogen administration group, and D shows a quantitative analysis result. The results show that each mouse in the blank control group has a certain amount of TH-positive cells (marked by arrows) in the substantia nigra; the number of TH-positive cells in the substantia nigra of each mouse in the solvent group decreases; and the number of TH-positive cells in the substantia nigra of each mouse in the plasminogen administration group is significantly higher than that in the solvent group. The results indicate that the plasminogen can restore TH-positive cells in the substantia nigra of Parkinson's model mice.

FIGS. 19A-C show immunohistochemical results of Iba-1 in the substantia nigra of Parkinson's model mice after 14 days of administration of plasminogen. A shows the blank control group, B shows the solvent PBS group, and C shows the plasminogen administration group. The results show that each mouse in the blank control group has a certain amount of microglia (marked by arrows) in the substantia nigra; the number of microglia in the substantia nigra of each mouse in the solvent control group is greater than that in the blank control group; and the number of microglia in the substantia nigra of each mouse in the plasminogen administration group is significantly less than that in the solvent control group, and close to that in the blank control group. The results indicate that the plasminogen can promote the recovery of microglia in the substantia nigra of Parkinson's model mice.

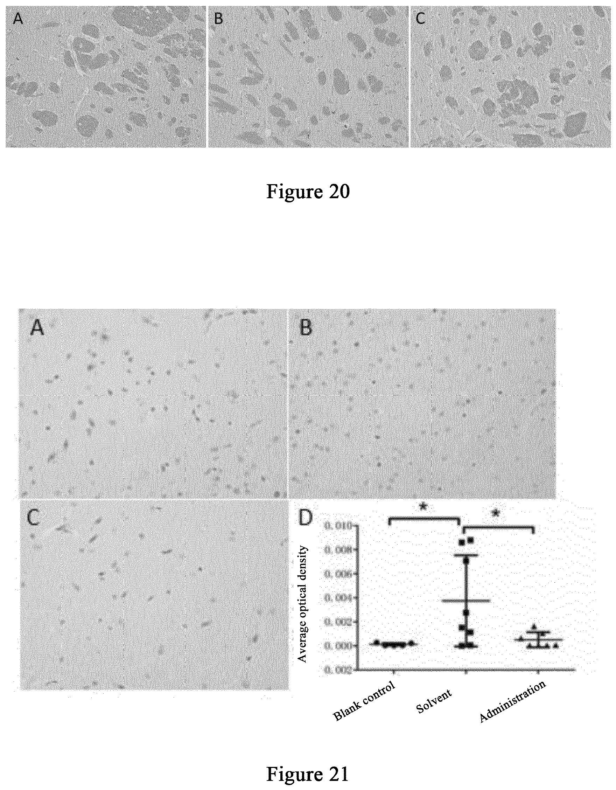

FIGS. 20A-C show LFB immunohistochemical results of the striatum in Parkinson's model mice after 14 days of administration of plasminogen. A shows the blank control group, B shows the solvent PBS group, and C shows the plasminogen administration group. The results show that each mouse in the blank control group has a certain amount of myelin sheaths in the striatum; the number of myelin sheaths in the striatum of each mouse in the solvent group is less than that in the blank control group; and the number of myelin sheaths in the striatum of each mouse in the plasminogen administration group is significantly greater than that in the solvent group. The results indicate that the plasminogen can promote the recovery of myelin sheaths of the striatum in Parkinson's model mice.

FIGS. 21A-D show immunohistochemical results of α-synuclein in the substantia nigra of Parkinson's model mice after 14 days of administration of plasminogen. A shows the blank control group, B shows the solvent group, C shows the plasminogen administration group, and D shows a quantitative analysis result of the average optical density. The results show that each mouse in the blank control group only has a small amount of α-synuclein in the substantia nigra; the amount of α-synuclein in the substantia nigra of each mouse in the solvent group is significantly higher than that in the blank control group (* indicates P<0.05); the amount of α-synuclein in the substantia nigra of each mouse in the plasminogen administration group is significantly less than that in the solvent group, and the statistical difference is significant (* indicates P<0.05); and the amount of α-synuclein in the substantia nigra of each mouse in the plasminogen administration group is close to that in the blank control group. This indicates that the plasminogen can reduce the expression of α-synuclein in the substantia nigra of Parkinson's model mice and improve the nerve damage degeneration.

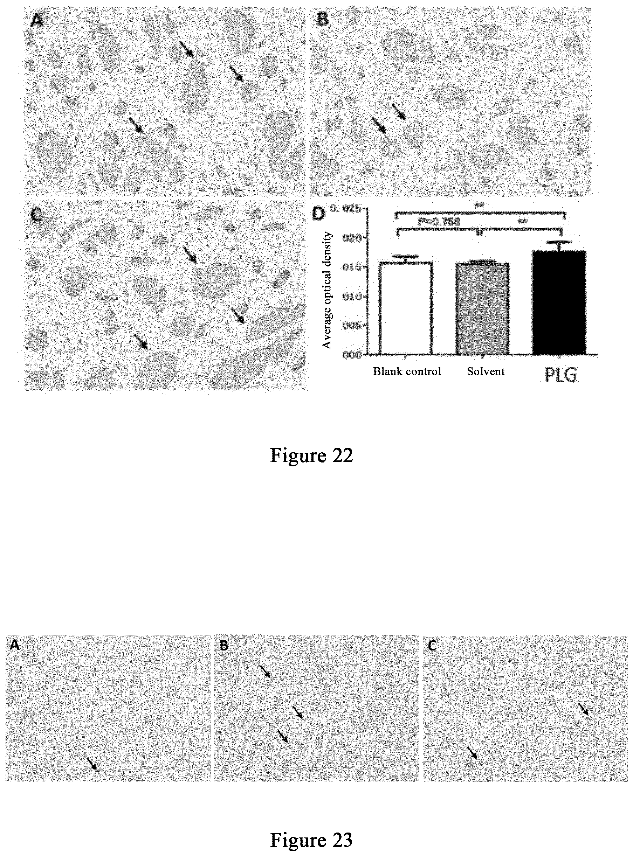

FIGS. 22A-D show immunohistochemical results of NF of the striatum in Parkinson's model mice after 14 days of administration of plasminogen. A shows the blank control group, B shows the solvent group, C shows the plasminogen administration group, and D shows a quantitative analysis result of the average optical density. The results show that each mouse in the blank control group has a certain amount of NF (marked by arrows) in the striatum; the number of NF in the striatum of each mouse in the solvent group is less than that in the blank control group; and the number of NF in the striatum of each mouse in the plasminogen administration group is significantly greater than that in the solvent group, and the statistical difference is extremely significant (** indicates P<0.01). The results indicate that the plasminogen can promote the recovery of NF expression of the striatum in Parkinson's model mice, and improve Parkinson's axonal damage of the striatum.

FIGS. 23A-C show immunohistochemical results of GFAP of the striatum in Parkinson's model mice after 14 days of administration of plasminogen. A shows the blank control group, B shows the solvent group, and C shows the plasminogen administration group. The results show that each mouse in the blank control group has a small amount of GFAP expressions (marked by arrows) in the striatum; the expression of GFAP in the striatum of each mouse in the solvent group is significantly higher than that in the blank control group; and the expression of GFAP in the striatum of each mouse in the plasminogen administration group is lower than that in the solvent group. This indicate that the plasminogen can reduce the GFAP expression in the striatum of Parkinson's model mice, and alleviate the damage of the striatum.

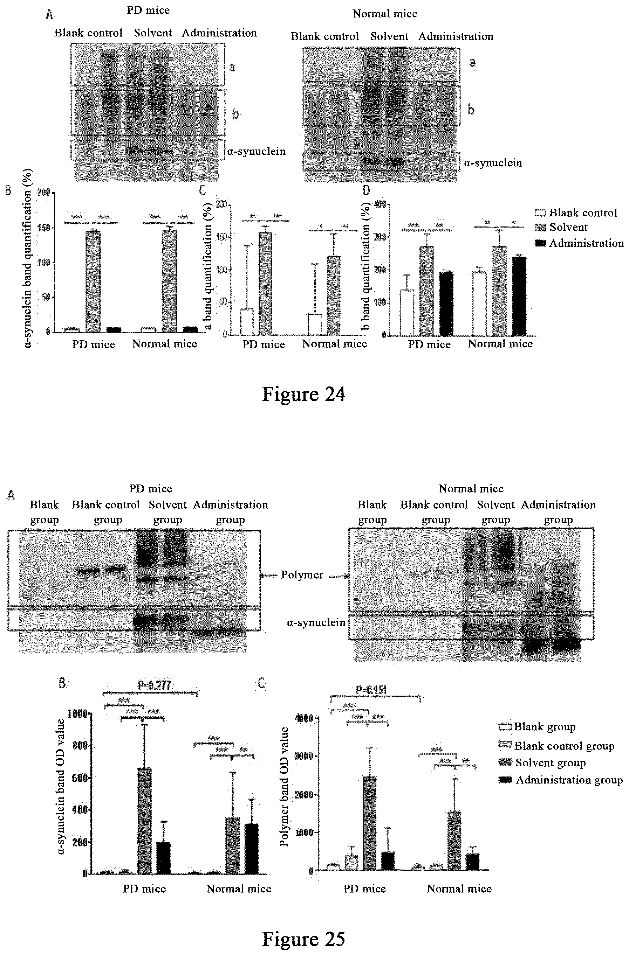

FIGS. 24A-D shows results of the effects of plasminogen on α-synuclein in cerebral homogenate of mice. A shows Tricine-page, and B, C, D show quantitative analysis results of band scanning of α-synuclein, a polymer a, and a polymer b, respectively. The results show that in the cerebral homogenate of Parkinson's model mice, the amount of α-synuclein in the plasminogen group is significantly lower than that in the solvent control group (*** represents P<0.001), and the amount of each of the polymers a and b is significantly lower than that in the solvent control group (** represents P<0.01, *** represents P<0.001); and in the cerebral homogenate of normal mice, the amount of α-synuclein in the plasminogen group is significantly lower than that in the solvent control group (*** represents P<0.001), and the amount of each of the polymers a and b is significantly lower than that in the solvent control group (* represents P<0.05, ** represents P<0.01). This indicates that the plasminogen can effectively promote the degradation of human α-synuclein and its polymers in the cerebral homogenates of Parkinson's model mice and normal mice.

FIGS. 25A-C show results of the effects of plasminogen on recombinant human α-synuclein in cerebral homogenate of mice. A shows a Western-blotting plot, and B and C show quantitative analysis results of band scanning of α-synuclein and a polymer, respectively. The results show that in the cerebral homogenate of Parkinson's model mice, the amount of α-synuclein in the plasminogen group is significantly lower than that in the solvent control group (** represents P<0.01), and the amount of the polymer is significantly lower than that in the solvent control group (*** represents P<0.001); and in the cerebral homogenate of normal mice, the amount of α-synuclein in the plasminogen group is significantly lower than that in the solvent control group (** represents P<0.01), and the amount of the polymer is significantly lower than that in the solvent control group (*** represents P<0.001). This indicates that the plasminogen can effectively promote the degradation of human α-synuclein and its polymer in the cerebral homogenates of Parkinson's model mice and normal mice.

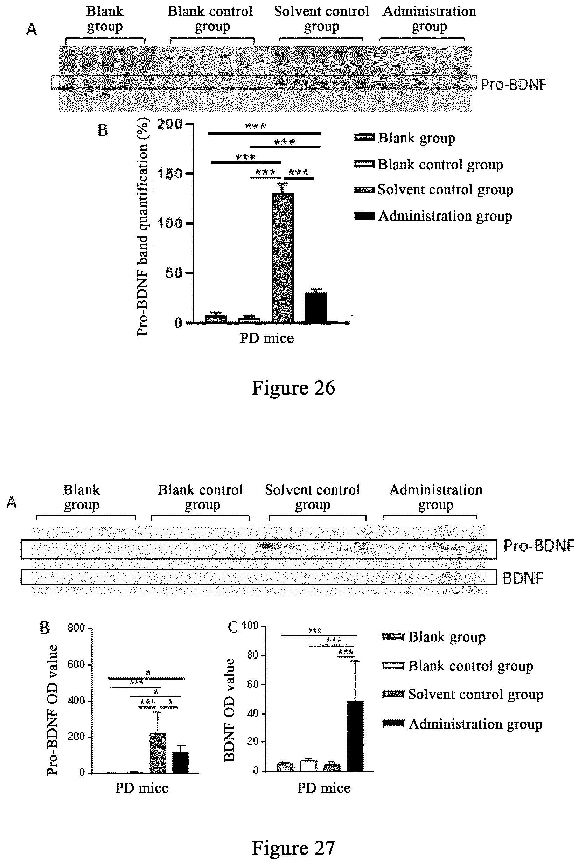

FIGS. 26A-B show the effects of plasminogen on recombinant human Pro-BDNF in cerebral homogenate of Parkinson's model mice, A shows an image of SDS-PAGE, and B shows a quantitative analysis result of SDS-PAGE bands. The results show that in the cerebral homogenate of Parkinson's model mice, the amount of Pro-BDNF in the plasminogen administration group is significantly lower than that in the solvent control group, and the difference is extremely significant (*** represents P<0.001). It indicates that the plasminogen can promote the cleavage of recombinant human Pro-BDNF in the cerebral homogenate of Parkinson's model mice.

FIGS. 27A-C show the effects of plasminogen on recombinant human Pro-BDNF in cerebral homogenate of Parkinson's model mice. A shows a Western blot image, B shows an analysis result of an optical density (OD) value of a Pro-BDNF band in Western blot, and C shows an analysis result of an optical density (OD) value of a BDNF band in the Western blot. The results show that in the cerebral homogenate of Parkinson's model mice, the amount of Pro-BDNF in the plasminogen administration group is significantly lower than that in the solvent control group, and the difference is significant (* represents P<0.05, *** represents P<0.001); and the amount of BDNF in the plasminogen administration group is significantly higher than that in the solvent control group, and the difference is extremely significant. It indicates that the plasminogen can promote the cleavage of recombinant human Pro-BDNF in the cerebral homogenate of Parkinson's model mice and the formation of mature BDNF.

DETAILED DESCRIPTION OF THE INVENTION

Fibrinolytic system is a system composed of a series of chemical substances involved in fibrinolysis. The chemical substances mainly include plasminogen, plasmin, plasminogen activators, and fibrinolysis inhibitors. The plasminogen activators include a tissue-type plasminogen activator (t-PA) and a urokinase-type plasminogen activator (u-PA). t-PA is a serine protease synthesized by vascular endothelial cells. t-PA activates plasminogen mainly on fibrin. The urokinase-type plasminogen activator (u-PA) is produced by renal tubular epithelial cells and vascular endothelial cells, and can directly activate plasminogen without the need for fibrin as a cofactor. Plasminogen (PLG) is synthesized in the liver. When blood coagulates, a large amount of PLG is adsorbed onto the fibrin network, and is activated to plasmin under the action of t-PA or u-PA to promote fibrinolysis. Plasmin (PL) is a serine protease, and has the following effects: degrading fibrin and fibrinogen; hydrolyzing a variety of blood coagulation factors such as V, VIII, X, VII, XI, and II; enabling plasminogen to be transformed into plasmin; hydrolyzing complements, etc. The fibrinolysis inhibitors include: plasminogen activator inhibitors (PAIs) and α2 antiplasmin (α2-AP). PAIs mainly include two types, i.e. PAI-1 and PAI-2, and can specifically bind to t-PA in a ratio of 1: 1 to inactivate t-PA and activate PLG at the same time. α2-AP is synthesized in the liver, and binds to PL in a ratio of 1: 1 to form a complex so as to inhibit the activity of PL. FXIII enables α2-AP to bind to fibrin in the form of covalent bond to attenuate the sensitivity of fibrin to the action of PL. Substances inhibiting the activity of the fibrinolytic system in vivo include: PAI-1, a complement C1 inhibitor, α2 antiplasmin, and an α2 macroglobulin.

The term "component of a plasminogen activation pathway" in the present invention covers:

1. plasminogen, Lys-plasminogen, Glu-plasminogen, micro-plasminogen, delta-plasminogen, and variants and analogs thereof;
2. plasmin and variants and analogs thereof; and
3. plasminogen activators, such as tPA, uPA, and a tPA or uPA variant or analog containing one or more domains (e.g. one or more kringle domains and proteolysis domains) of tPA or uPA.

The above "variants" of plasminogen, plasmin, tPA, and uPA include all naturally occurring human genetic variants and other mammalian forms of these proteins, and a protein that is obtained by adding, deleting and/or substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid and still has the activity of plasminogen, plasmin, tPA or uPA. For example, the "variants" of plasminogen, plasmin, tPA, and uPA include mutational variants of these proteins that are obtained by substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid with conservative amino acids.

The "plasminogen variants" of the present invention include proteins having at least 75%, 80%, 85%, 90%, 95%, 96%, 97%, 98% or 99% sequence identity with sequence 2, 6, 8, 10 or 12 and still having the activity, such as the lysine binding activity and the proteolytic activity, of plasminogen. For example, the "plasminogen variants" of the present invention may be proteins that are obtained by adding, deleting and/or substituting 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid on the basis of sequence 2, 6, 8, 10 or 12 and still have the activity, such as the lysine binding activity and the proteolytic activity, of plasminogen. Specifically, the plasminogen variants of the present invention include all naturally occurring human genetic variants and other mammalian forms of these proteins, and mutational variants of these proteins that are obtained by substituting, for example, 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid with conservative amino acids.

The plasminogen of the present invention may be a human plasminogen ortholog from a primate or a rodent, or a variant thereof that retains the activity, such as the lysine binding activity and the proteolytic activity, of plasminogen, such as plasminogen shown as sequence 2, 6, 8, 10 or 12, and human natural plasminogen shown as sequence 2.

The above "analogs" of plasminogen, plasmin, tPA, and uPA include compounds respectively providing functions basically similar to those of plasminogen, plasmin, tPA, or uPA.

The above "variants" and "analogs" of plasminogen, plasmin, tPA, and uPA include "variants" and "analogs" containing one or more domains (e.g. one or more kringle domains and proteolysis domains) of plasminogen, plasmin, tPA, and uPA. For example, the "variants" and "analogs" of plasminogen include plasminogen variants and analogs containing one or more domains (e.g. one or more kringle domains and proteolysis domains) of plasminogen, such as mini-plasminogen. The "variants" and "analogs" of plasmin include plasmin "variants" and "analogs" containing one or more domains (e.g. one or more kringle domains and proteolysis domains) of plasmin, such as mini-plasmin and delta-plasmin (δ-plasmin).

Whether the above "variants" or "analogs" of plasminogen, plasmin, tPA or uPA have the activity of plasminogen, plasmin, tPA or uPA, or whether the above "variants" or "analogs" of plasminogen, plasmin, tPA or uPA respectively provides functions basically similar to those of plasminogen, plasmin, tPA or uPA can be detected by the methods known in the art. For example, the activity of activated plasmin is determined by enzymography, an enzyme-linked immunosorbent assay (ELISA) or fluorescence-activated cell sorting (FACS), or determined by the methods described in the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest. 74 (5): 1625-33; Gravanis I, Tsirka SE (February 2008). "Tissue-type plasminogen activator as a therapeutic target in stroke". Expert Opinion on Therapeutic Targets. 12 (2): 159-70; and Geiger M, Huber K, Wojta J, Stingl L, Espana F, Griffin JH, Binder BR (Aug 1989). "Complex formation between urokinase and plasma protein C inhibitor in vitro and in vivo". Blood. 74 (2): 722-8.

In some embodiments of the present invention, the "component of the plasminogen activation pathway" of the present invention is plasminogen. In some embodiments, the plasminogen is human full-length plasminogen or a conservatively substituted variant thereof that retains the activity (e.g., the lysine binding activity and the proteolytic activity) of plasminogen. In some embodiments, the plasminogen is selected from the group consisting of Glu-plasminogen, Lys-plasminogen, small plasminogen, microplasminogen, delta-plasminogen or variants thereof that retain the activity (e.g. the lysine binding activity and the proteolytic activity) of plasminogen. In some embodiments, the plasminogen is natural or synthesized human plasminogen, or a conservatively substituted variant or fragment thereof that retains the activity (e.g. the lysine binding activity and the proteolytic activity) of plasminogen. In some embodiments, the plasminogen is a human plasminogen ortholog from a primate or a rodent, or a conservatively substituted variant or fragment thereof that retains the activity of plasminogen. In some embodiments, the plasminogen contains an amino acid sequence shown as sequence 2, 6, 8, 10 or 12. In some embodiments, the plasminogen contains a conservatively substituted sequence of the amino acid sequence shown as sequence 2, 6, 8, 10 or 12. In some embodiments, the amino acid of the plasminogen is shown in sequence 2, 6, 8, 10 or 12. In some embodiments, the plasminogen is a conservatively substituted variant of plasminogen shown as sequence 2, 6, 8, 10 or 12. In some embodiments, the plasminogen is human natural plasminogen or a conservative mutant thereof. In some embodiments, the plasminogen is human natural plasminogen shown as sequence 2 or a conservatively substituted variant thereof.

A "compound capable of directly activating plasminogen or indirectly activating plasminogen by activating an upstream component of a plasminogen activation pathway" refers to any compound that can directly activate plasminogen or indirectly activate plasminogen by activating an upstream component of a plasminogen activation pathway, such as tPA, uPA, streptokinase, saruplase, alteplase, reteplase, tenecteplase, anistreplase, monteplase, lanoteplase, pamiteplase, and staphylokinase.

An "antagonist of a fibrinolysis inhibitor" of the present invention is a compound that antagonizes, weakens, blocks, or prevents the action of a fibrinolysis inhibitor. The fibrinolysis inhibitor is, for example, PAI-1, a complement C1 inhibitor, $\alpha2$ antiplasmin or an $\alpha2$ macroglobulin. The antagonist is, for example, an antibody of PAI-1, a complement C1 inhibitor, $\alpha2$ antiplasmin or an $\alpha2$ macroglobulin, or antisense RNA or small RNA that blocks or down-regulates the expression of PAI-1, a complement C1 inhibitor, $\alpha2$ antiplasmin or an $\alpha2$ macroglobulin, or a compound that occupies a binding site of PAI-1, a complement C1 inhibitor, $\alpha2$ antiplasmin or an $\alpha2$ macroglobulin and does not have functions of PAI-1, a complement C1 inhibitor, $\alpha2$ antiplasmin or an $\alpha2$ macroglobulin, or compound that blocks a binding domain and/or an activity domain of PAI-1, a complement C1 inhibitor, $\alpha2$ antiplasmin or an $\alpha2$ macroglobulin.

Plasmin is a key component of a plasminogen activation (PA) system. It is a broad-spectrum protease, and can hydrolyze several components, including fibrin, gelatin, fibronectin, laminin, and proteoglycans, of an extracellular matrix (ECM). In addition, plasmin can activate some matrix metalloproteinase precursors (pro-MMPs) to active matrix metalloproteinases (MMPs). Therefore, plasmin is considered as an important upstream regulator of extracellular proteolysis. Plasmin is formed by proteolysis of plasminogen with two types of physiological PAs, i.e. a tissue-type plasminogen activator (tPA) and a urokinase-type plasminogen activator (uPA). Due to relatively high levels of plasmin in plasma and other body fluids, it has traditionally been thought that the regulation of the PA system is mainly achieved through the synthesis and activity levels of PAs. The synthesis of components of the PA system is strictly regulated by different factors, such as a hormone, a growth factor, and a cytokine. In addition, there are specific physiological inhibitors of plasmin and PAs. A main inhibitor of plasmin is $\alpha2$-antiplasmin. The activity of PAs is regulated by both of a plasminogen activator inhibitor 1 (PAI-1) for inhibiting uPA and tPA and a plasminogen activator inhibitor 2 (PAI-2) for mainly inhibiting uPA. There are uPA-specific cell surface receptors (uPARs) having the direct hydrolysis activity on the surface of some cells.

Plasminogen is a single-stranded glycoprotein, is composed of 791 amino acids, and has a molecular wight of about 92 kDa. Plasminogen is mainly synthesized in the liver, and is abundant in the extracellular fluid. The plasminogen content in plasma is about 2 $\mu$M. Therefore, plasminogen is a huge potential source of the proteolytic activity in tissues and body fluids. Plasminogen is present in two molecular forms, i.e. glutamate-plasminogen (Glu-plasminogen) and lysine-plasminogen (Lys-plasminogen). Naturally secreted and uncleaved plasminogen has an amino-terminal (N-terminal) glutamate, so it is referred to as glutamate-plasminogen. However, glutamate-plasminogen is hydrolyzed to lysine-plasminogen at Lys76-Lys77 in the presence of plasmin. Compared with glutamate-plasminogen, lysine-plasminogen has higher affinity to fibrin and can be activated by PAs at a higher rate. Arg560-Val561 peptide bonds of the two forms of plasminogen can be cleaved by uPA or tPA to form double-stranded protease plasmin linked via a disulfide bond. The amino-terminal moiety of plasminogen contains five homologous tri-circles, i.e. kringles; and the carboxyl-terminal moiety of plasminogen contains protease domains. Some kringles contain lysine binding sites for mediating specific interaction of plasminogen and fibrin, as well as its inhibitor $\alpha2$-AP. It is found recently that a plasminogen fragment of 38 kDa that contains kringles 1 to 4 is an effective inhibitor for angiogenesis. This fragment is named angiostatin, which can be produced by hydrolyzing plasminogen with several proteases.

A main substrate of plasmin is fibrin, and the dissolution of fibrin is a key for preventing pathological thrombosis. Plasmin also has substrate specificity to several components, including laminin, fibronectin, proteoglycans, and gelatin, of ECM, suggesting that plasmin also plays an important role in reconstruction of ECM. Indirectly, plasmin can also degrade other components, including MMP-1, MMP-2, MMP-3, and MMP-9, of ECM by transforming some protease precursors into active proteases. Therefore, it has been proposed that plasmin may be an important upstream regulator of extracellular proteolysis. In addition, plasmin has the ability to activate certain potential growth factors. In vitro, plasmin can also hydrolyze components of a complement system and release chemotactic complement fragments.

"Plasmin" is a very important enzyme present in the blood, which can hydrolyze a fibrin clot to fibrin degradation products and D-dimer.

"Plasminogen" is a zymogen form of plasmin. According to sequences in Swiss Prot, a glycoprotein composed of 810 amino acids, having a molecular weight of about 90 kDa, mainly synthesized in the liver, and capable of circulating in the blood is calculated based on an amino acid sequence (sequence 4) of natural human plasminogen containing a signal peptide, and a cDNA sequence for encoding the amino acid sequence is shown as sequence 3. Full-length plasminogen contains seven domains, i.e. a serine protease domain at the C terminus, a Pan Apple (PAp) domain at the N terminus, and five Kringle domains (Kringle1 to Kringle5). Referring to sequences in Swiss Prot, the signal peptide includes residues Met1-Gly19, PAp includes residues Glu20-Val98, Kringle1 includes residues Cys103-Cys181, Kringle2 includes residues Glu184-Cys262, Kringle3 includes residues Cys275-Cys352, Kringle4 includes residues Cys377-Cys454, and Kringle5 includes residues Cys481-Cys560. According to data of NCBI, the serine protease domain includes residues Val581-Arg804.

Glu-plasminogen is human natural full-length plasminogen and is composed of 791 amino acids (without a signal peptide of 19 amino acids), a cDNA sequence for encoding the sequence is shown as sequence 1, and an amino acid sequence of Glu-plasminogen is shown as sequence 2. In vivo, there is Lys-plasminogen formed by hydrolyzing Glu-plasminogen at amino acids at the 76th site and the 77th site, which is shown as sequence 6, and a cDNA sequence for encoding the amino acid sequence is shown as sequence 5. Delta-plasminogen (δ-plasminogen) is full-length plasminogen lacking a fragment from Kringle2 to Kringle5 and containing only Kringle1 and a serine protease domain (also referred to as a protease domain (PD)), an amino acid sequence (sequence 8) of delta-plasminogen has been reported in a document, and a cDNA sequence for encoding the amino acid sequence is shown as sequence 7. Mini-plasminogen is composed of Kringle5 and a serine protease domain, it has been reported in a document that an amino acid sequence of mini-plasminogen includes residues Val443-Asn791 (taking a Glu residue in a sequence of Glu-plasminogen without a signal peptide as the starting amino acid), and is shown as sequence 10, and a cDNA sequence for encoding the amino acid sequence is shown as sequence 9. Micro-plasminogen contains only a serine protease domain, it has been reported in a document that an amino acid sequence of micro-plasminogen includes residues Ala543-Asn791 (taking a Glu residue in a sequence of Glu-plasminogen without a signal peptide as the starting amino acid), and it has also been reported in the patent document CN102154253A that the sequence of micro-plasminogen includes residues Lys531-Asn791 (taking a Glu residue in a sequence of Glu-plasminogen without a signal peptide as the starting amino acid). In this patent application, the amino acid sequence of micro-plasminogen is referred to the patent document CN102154253A, and is shown as sequence 12, and a cDNA sequence for encoding the amino acid sequence is shown as sequence 11.

The structure of full-length plasminogen is also described in the paper of Aisina, et al. (Aisina R B, Mukhametova L I. Structure and function of plasminogen/plasmin system [J]. Russian Journal of Bioorganic Chemistry, 2014, 40 (6): 590-605). In this paper, Aisina, et al. describe that plasminogen includes Kringle1, 2, 3, 4, and 5 domains and a serine protease domain (also referred to as a protease domain (PD)). Kringles are responsible for binding plasminogen to ligands having low molecular wights and high molecular weights (i.e. the lysine binding activity), so that plasminogen is transformed into a more open conformation, which can be activated more easily. The protease domain (PD) includes residues Val562-Asn791, and tPA and uPA specifically cleave an activation bond at sites Arg561-Val562 in plasminogen to transform plasminogen into plasmin. Therefore, the protease domain (PD) is a region giving the proteolytic activity of plasminogen. Herein, the terms "plasmin", "fibrinolysin", and "fibrinolytic enzyme" are interchangeable and have the same meaning. The terms "plasminogen", "profibrinolysin", and "fibrinolytic zymogen" are interchangeable and have the same meaning.

In the present invention, the "plasminogen deficiency" means that the plasminogen content or activity in a subject is less than that in a normal person, and is low enough to affect normal physiological functions of the subject. The "plasminogen deficiency" means that the plasminogen content or activity in a subject is less than that in a normal person, the activity or expression of plasminogen is extremely low, and normal physiological functions can only be maintained by providing exogenous plasminogen.

Those in the art may understand that all technical solutions of plasminogen of the present invention are applicable to plasmin, and thus the technical solutions described in the present invention cover plasminogen and plasmin. During circulation, plasminogen is in a closed inactive conformation, and is transformed into activate plasmin in an open conformation under the mediation of a plasminogen activator (PA) when binding to a thrombus or cell surface. Active plasmin can further hydrolyze a fibrin clot to fibrin degradation products and D-dimer, so as to dissolve a thrombus. The PAp domain of plasminogen contains an important determinant for maintaining plasminogen in a closed inactive conformation, and the KR domain of plasminogen can bind to a lysine residue present in a receptor and a substrate. A variety of known enzymes that can be used as plasminogen activators include: a tissue-type plasminogen activator (tPA), a urokinase-type plasminogen activator (uPA), a kallikrein, a blood coagulation factor XII (Hageman factor), etc.

A "plasminogen active fragment" refers to a fragment having the activity of binding to lysine in a target sequence of a substrate (the lysine binding activity), or the activity of exerting proteolytic function (the proteolytic activity), or the proteolytic activity and the lysine binding activity. The technical solutions related to plasminogen of the present invention cover a technical solution of replacing plasminogen with a plasminogen active fragment. In some embodiments, the plasminogen active fragment of the present invention contains the serine protease domain of plasminogen or is composed of the serine protease domain of plasminogen. In some embodiments, the plasminogen active fragment of the present invention contains sequence 14, or contains an amino acid sequence having at least 80%, 90%, 95%, 96%, 97%, 98% or 99% identity with sequence 14, or is composed of sequence 14, or is composed of the amino acid sequence having at least 80%, 90%, 95%, 96%, 97%, 98% or 99% identity with sequence 14. In some embodiments, the plasminogen active fragment of the present invention contains one or more regions selected from Kringle1, Kringle2, Kringle3, Kringle4, and Kringle5 or conservatively substituted variants thereof, or is composed of one or more regions selected from Kringle1, Kringle2, Kringle3, Kringle4, and Kringle5 or conservatively substituted variants thereof. In some embodiments, the plasminogen of the present invention includes a protein containing the above plasminogen active fragment. At present, assays of plasminogen in the blood and its activity include: an tissue-type plasminogen activator activity assay (t-PAA), a plasma tissue-type plasminogen activator antigen assay (t-PAAg), a plasma tissue-type plasminogen activity assay (plgA), a plasma tissue plasminogen antigen assay (plgAg), a plasma tissue-type plasminogen activator inhibitor activity assay, a plasma tissue-type plasminogen activator inhibitor antigen assay, and a plasma plasmin-antiplasmin complex assay (PAP). The most commonly used test method is a chromogenic substrate method: streptokinase (SK) and a chromogenic substrate are added to plasma to be tested, PLG in the plasma to be tested is transformed into PLM under the action of SK, PLM acts on the chromogenic substrate, absorbance is measured by using a spectrophotometer, and increase in absorbance is proportional to the activity of plasminogen. In addition, immunohistochemistry, gel electrophoresis, immunoturbidimetry, radial immunodiffusion, etc. can also be adopted to test the activity of plasminogen in the blood.

An "ortholog" refers to a homolog of different species, includes a protein homolog and a DNA homolog, and is also referred to as a vertical homolog. It specifically refers to a protein or a gene in different species that has evolved from the same ancestral gene. The plasminogen of the present invention includes human natural plasminogen, and also includes plasminogen orthologs derived from different species and having the activity of plasminogen.

A "conservatively substituted variant" refers to that a given amino acid residue is changed, but the whole conformation and function of a protein or enzyme are not changed. For example, an amino acid in an amino acid sequence of a parent protein is substituted with an amino acid with similar properties (e.g., acidity, alkalinity, and hydrophobicity). The amino acid with similar properties is well known. For example, arginine, histidine, and lysine are hydrophilic alkaline amino acids and can be substituted with each other. Similarly, isoleucine is a hydrophobic amino acid and can be substituted with leucine, methionine or valine. Therefore, the similarity of amino acid sequences of two protein having similar functions may be different. For example, 70% to 99% similarity (identity) based on the MEGALIGN algorithm The "conservatively substituted variant" also includes a polypeptide or an enzyme that has more than 60% amino acid sequence identity determined based on BLAST or FASTA algorithm, preferably, more than 75% identity, more preferably, more than 85% identity, and the most preferably, more than 90% identity. The polypeptide or the enzyme has the same or basically similar properties or function compared to a natural or parent protein or enzyme.

"Isolated" plasminogen refers to a plasminogen protein isolated and/or recovered from a natural environment of plasminogen. In some embodiments, the plasminogen is purified (1) to the purity (by weight) of more than 90%, more than 95% or more than 98%, such as more than 99% determined by the Lowry method, (2) to an extent sufficient to obtain at least 15 residues at the N terminus or in an internal amino acid sequence by using a rotary cup sequencer, or (3) to homogeneity that is determined by sodium dodecyl sulfate-polyacrylamide gel electrophoresis (SDS-PAGE) using Coomassie blue or silver staining under reducing or non-reducing conditions. The isolated plasminogen also includes plasminogen prepared from a recombinant cell by a bioengineering technology and isolated by at least one purification step.

Herein, the terms "polypeptide", "peptide", and "protein" are interchangeable, refer to an aggregation form of amino acids of any length, and may include genetically encoded and non-genetically encoded amino acids, chemically or biogeochemically modified or derived amino acids, and a polypeptide having a modified peptide backbone. The terms include fusion proteins, which include, but are not limited to, a fusion protein having an heterogenous amino acid sequence, a fusion having heterogenous and homologous leader sequences (having or without an N-terminal methionine residue), etc.

"Amino acid sequence identity percentage (%)" relative to a reference polypeptide sequence is defined as, after gaps have been introduced as necessary to achieve the maximum percentage sequence identity, and no conservative substitutions are considered as a part of the sequence identity, the percentage of amino acid residues, which are identical to amino acid residues in the reference polypeptide sequence, in a candidate sequence. Comparison for determining percentage amino acid sequence identity can be achieved in a variety of ways within the technical scope of the art. For example, software available to the public, such as BLAST, BLAST-2, ALIGN, and Megalign (DNASTAR), is adopted. Those skilled in the art can determine appropriate parameters used for comparing sequences, such as any algorithm needed to achieve the maximum comparison over the full lengths of sequences to be compared. However, for the purpose of the present invention, an amino acid sequence identity percentage value is generated by using the sequence comparison computer program ALIGN-2.

In a case that ALIGN-2 is adopted to compare amino acid sequences, % amino acid sequence identity of a given amino acid sequence A relative to a given amino acid sequence B (or may be expressed as that the given amino acid sequence A has or contains certain % amino acid sequence identity relative to, with, or to the given amino acid sequence B) is calculated as follows:

$$X/Y \times 100\%$$

where, X is the number of amino acid residues, identically matched with amino acid residues in B, in A that is determined by the sequence comparison program ALIGN-2, and Y is the total number of amino acid residues in B. It is to be understood that in a case that the length of the amino acid sequence A differs from that of the amino acid sequence B, % amino acid sequence identity of A relative to B is not equal to % amino acid sequence identity of B relative to A. Unless otherwise specifically described, all % amino acid sequence identity values used herein are obtained by using the ALIGN-2 computer program as described in the preceding paragraph.

As used herein, the term "treatment" refers to obtaining a desired pharmacological and/or physiological effect. The effect may be complete or partial prevention of occurrence and onset of a disease or its symptoms, partial or complete alleviation of a disease and/or its symptoms, and/or partial or complete cure of a disease and/or its symptoms, which includes: (a) prevention of occurrence or onset of a disease in a subject who may have a predisposition to the disease but has not been diagnosed with the disease; (b) inhibition of a disease, i.e. retardation of the formation of the disease; and (c) alleviation of a disease and/or its symptoms, i.e. subsidence or disappearance of the disease and/or its symptoms.

Herein, the terms "individual", "subject", and "patient" are interchangeable, and refer to a mammal, which includes, but is not limited to, murine (rats and mice), non-human primates, humans, dogs, cats, ungulates (e.g. horses, cattle, sheep, pigs, and goats), etc.

A "therapeutically effective amount" or "effective dose" refers to an amount of a component of a plasminogen activation pathway or its related compound (e.g. plasminogen) that is sufficient to prevent and/or treat a disease when administered to a mammal or other subjects to treat the disease. The "therapeutically effective amount" is changed with the component of the plasminogen activation pathway or its related compound (e.g. plasminogen) used, a disease of a subject to be treated and/or the severity of symptoms, age, and weight, etc.

Preparation of the plasminogen of the present invention

Plasminogen can be isolated from nature and purified for further therapeutic use, or can be synthesized by a standard chemical peptide synthesis technology. In a case that a polypeptide is synthesized chemically, plasminogen can be synthesized from a liquid phase or a solid phase. Solid-phase polypeptide synthesis (SPPS) (in which a C-terminal amino acid of a sequence is attached to an insoluble support, followed by sequential addition of the remaining amino acids in the sequence) is a method suitable for chemical synthesis of plasminogen. Various SPPS methods, such as Fmoc and Boc, can be used to synthesize plasminogen. The solid-phase synthesis technique is described in Barany, et al. Solid-Phase Peptide Synthesis; The Peptides: Analysis, Synthesis, Biology. Vol. 2: Special Methods in Peptide Synthesis, Part A, 3-284; Merrifield. Solid Phase Peptide Synthesis. I. The Synthesis of a Tetrapeptide, et al., J. Am. Chem. Soc., 85: 2149-2156 (1963); Stewart, et al. Solid Phase Peptide Synthesis, 2nd ed. Pierce Chem. Co., Rockford, Ill. (1984); Ganesan A. 2006 Mini Rev. Med Chem. 6: 3-10; and Camarero JA, et al. 2005 Protein Pept Lett. 12: 723-8. In short, small insoluble porous beads are treated with a functional unit on which a peptide chain is built. After recirculation of coupling/deprotection, the attached solid-phase free N-terminal amine is coupled to a single N-protected amino acid unit. This unit is then deprotected to reveal a new N-terminal amine that can be attached to another amino acid. The peptide remains immobilized on the solid phase and then is cleaved.

Plasminogen of the present invention can be produced by a standard recombination method. For example, a nucleic acid for encoding plasminogen is inserted into an expression vector so as to be operably connected to a regulatory sequence in the expression vector. The expression regulatory sequence includes, but is not limited to, a promoter (e.g., a naturally related or heterogenous promoter), a signal sequence, an enhancer element, and a transcription termination sequence. The expression regulatory sequence may be a eukaryotic promoter in the vector, and the vector can transform or transfect eukaryotic host cells (e.g., COS or CHO cells). Once the vector is incorporated into a suitable host, the vector maintains the host under conditions suitable for high expression of a nucleotide sequence and collection and purification of plasminogen.

The suitable expression vector usually replicates in the host organism as an episome or an integrated part of the host chromosomal DNA. Normally, the expression vector contains a selectable marker (e.g., ampicillin resistance, hygromycin b resistance, tetracycline resistance, kanamycin resistance, and neomycin resistance) to facilitate detection of cells transformed with an exogenous desired DNA sequence.

Exemplary prokaryotic host cells that can be used to clone a polynucleotide for encoding a subject antibody include Escherichia coli. Other suitable microbial hosts include: Bacillus such as Bacillus subtilis, and other Enterobacteriaceae such as Salmonella, Serratia, and various Pseudomonas species. Expression vectors can also be generated in these prokaryotic hosts, and usually contain expression control sequences (origin of replication) compatible with the host cells. In addition, there are many known promoters, such as a lactose promoter system, a tryptophan (trp) promoter system, a beta-lactamase promoter system, and a promoter system from bacteriophage $\lambda$. The promoter usually controls expression optionally in a sequence of an operator gene, and has a ribosome binding site sequence for initiating and completing transcription and translation.

Other microorganisms, such as yeast, can also be used for expression. Exemplary suitable yeast host cells include yeast (e.g., Saccharomyces cerevisiae (S. cerevisiae)) and Pichia, and the suitable vector has an expression control sequence (e.g., a promoter), origin of replication, a terminator sequence, etc. according to the requirements. Typical promoters contain 3-phosphoglycerate kinase and other glycogenolysis enzymes. Inducible yeast promoters specially include promotes from alcohol dehydrogenase, hetero-cytochrome C, and enzymes responsible for using maltose and galactose.

In addition to microorganisms, mammalian cells (e.g., mammalian cells cultured in a cell culture medium in vitro) can also be used for expressing and generating the anti-Tau antibody (e.g., a polynucleotide for encoding a subject anti-Tau antibody) of the present invention. Referring to Winnacker, From Genes to Clones, VCH Publishers, N.Y., N.Y. (1987). Suitable mammal host cells include a CHO cell line, various Cos cell line, HeLa cells, a myeloma cell line, and transformed B cells or hybridoma. An expression vector used for these cells may include an expression control sequence such as origin of replication, a promoter, and an enhancer (Queen, et al. Immunol. Rev. 89: 49 (1986)), and a necessary processing information site such as a ribosome binding site, an RNA splicing site, a polyadenylation site, and a transcription terminator sequence. Exemplary suitable expression control sequences include derived promoters such as a white immunoglobulin gene, SV40, an adenovirus, a bovine papillomavirus, and a cytomegalovirus. Referring to Co, et al. J. Immunol. 148: 1149 (1992).

Once the plasminogen of the present invention is synthesized (by the chemical or recombination method), the plasminogen of the present invention is purified in accordance with the standard procedure in the art, which includes ammonium sulfate precipitation, affinity column chromatography, high performance liquid chromatography (HPLC), gel electrophoresis, etc. The plasminogen is substantially pure, for example, at least about 80% to 85% pure, at least 85% to 90% pure, at least about 90% to 95% pure, 98% to 99% pure or purer. For example, the plasminogen does not contain contaminants such as cellular debris and macromolecules other than the target product.

Drug preparation

A therapeutic preparation is a lyophilized preparation or an aqueous solution formed by mixing a component of a plasminogen activation pathway or its related compound (e.g. plasminogen) with required purity with an optional pharmaceutical carrier, an excipient or a stabilizer (Remington's Pharmaceutical Sciences, 16th Edition, Osol, A. ed. (1980)). The acceptable carrier, excipient or stabilizer at a used dose and concentration is nontoxic to subjects, and include a buffer such as phosphates, citrates, and other organic acids; an antioxidant such as ascorbic acid and methionine; a preservative (e.g. octadecyl dimethyl benzyl ammonium chloride, hexanediamine chloride, benzalkonium chloride, benzethonium chloride, phenol, butanol, benzyl alcohol, alkyl parabens such as methyl and ethyl parabens, catechol, resorcinol, cyclohexanol, 3-pentanol, and m-cresol); a polypeptide with a low molecular weight (less than about 10 residues); a protein such as serum albumin, gelatin, and immunoglobulin; a hydrophilic polymer such as polyvinylpyrrolidone; an amino acid such as glycine, glutamine, asparagine, histidine, arginine, and lysine; monosaccharide, disaccharide, and other carbohydrates such as glucose, mannose, and dextrin; a chelant such as EDTA; saccharides such as sucrose, mannitol, fucose, and sorbitol; salt-forming counterions such as sodium; a metal complex (e.g. a zinc-protein complex); and/or a non-ionic surfactant such as TWEEN™, PLURONICS™, and polyethylene glycol (PEG). A preferred lyophilized anti-VEGF antibody preparation is described in WO 97/04801, which is incorporated herein by reference.

The preparation of the present invention may also contain more than one active compound needed for treating a specific symptom, and preferably, the active compounds are complementary and do not have side effects on each other.

The plasminogen of the present invention can be encapsulated in a microcapsule prepared by techniques such as coacervation or interfacial polymerization, for example, can be placed in a colloidal drug delivery system (e.g. a liposome, an albumin microsphere, a microemulsion, nanoparticles, and a nanocapsule) or placed in hydroxymethylcellulose in a macroemulsion or a gel-microcapsule and a poly-(methyl methacrylate) microcapsule. These techniques are disclosed in Remington's Pharmaceutical Sciences 16th edition, Osol, A. Ed. (1980).

The component of the plasminogen activation pathway or its related compound (e.g., plasminogen) used for in vivo administration needs to be sterile. It can be easily achieved by filtration with a sterile filter before or after lyophilization and re-preparation.

The component of the plasminogen activation pathway or its related compound (e.g., plasminogen) of the present invention can be used for preparation of a sustained-release preparation. Exemplary suitable sustained-release preparations include semipermeable matrices of solid hydrophobic polymers having a shape and containing glycoproteins, such as membranes or microcapsules. Exemplary sustained-release matrices include polyesters, hydrogels (e.g., poly(2-hydroxyethyl-methacrylate) (Langer, et al. J. Biomed. Mater. Res., 15: 167-277 (1981); Langer, Chem. Tech., 12: 98-105 (1982)) or polyvinyl alcohol, polylactide (US patent 3773919, EP 58,481), a copolymer of L-glutamic acid andγethyl-L-glutamic acid (Sidman, et al. Biopolymers 22: 547 (1983)), non-degradable ethylene-vinyl acetate (Langer, et al. same as above) or degradable lactic acid-glycolic acid copolymer such as Lupron Depot™ (an injectable microsphere composed of a lactic acid-glycolic acid copolymer and leuprolide acetate), and poly-D-(-)-3-hydroxybutyric acid. The compounds, such as ethylene-vinyl acetate and lactic acid-glycolic acid, can sustainably release molecules for more than 100 days, and some hydrogels release proteins for a short time period. Rational strategies for stabilizing proteins can be designed based on the relevant mechanisms. For example, in a case that the mechanism of coacervation is formation of intermolecular S-S bonds through the exchange of thiodisulfide bonds, proteins can be stabilized by modifying sulfhydryl residues, lyophilizing from an acid solution, controlling humidity, using an appropriate additive, and developing a specific polymer matrix composition.

Administration and dosage

The pharmaceutical composition of the present invention can be administered by different methods, such as nasal inhalation, aerosol inhalation, nasal drops or eye drops, an intravenous method, an intraperitoneal method, a subcutaneous method, an intracranial method, an intrathecal method, an intraarterial method (e.g. via the carotid artery), an intramuscular method, and a rectal administration.

Preparations for parenteral administration include sterile aqueous or non-aqueous solutions, suspensions, and emulsions. Non-aqueous solvents include propylene glycol, polyethylene glycol, vegetable oils such as olive oil, and injectable organic esters such as ethyl oleate. Aqueous vectors include water, alcoholic/aqueous solutions, emulsions, and suspensions such as saline and a buffer medium. Parenteral intermedia include a sodium chloride solution, Ringer's dextrose, dextrose, sodium chloride, and fixed oils. Intravenous intermedia include fluids and nutritional supplements, electrolyte supplements, etc. A preservative and other additives, such as an antimicrobial, an antioxidant, a chelator, and inert gas, may be present.

Medical staffs will determine a dosage regimen based on various clinical factors. As well known in the medical field, a dosage regimen for any patient is determined according to a variety of factors, including the body size of a patient, the body surface area, age, a specific compound to be administered, sex, the frequency and path of administration, general health conditions, and other drugs to be administered together. A daily dosage range of the pharmaceutical composition containing plasminogen of the present invention may be, for example, about 0.0001-2000 mg/kg, or about 0.001-500 mg/kg (e.g. 0.02 mg/kg, 0.25 mg/kg, 0.5 mg/kg, 0.75 mg/kg, 10 mg/kg, and 50 mg/kg) of body weight of a subject. For example, a dose may be 1 mg/kg of body weight or 50 mg/kg of body weight, or within a range of 1-50 mg/kg of body weight, or at least 1 mg/kg of body weight. Doses greater or less than these exemplary ranges are also covered, especially in view of the above factors. Intermediate doses within the above ranges also fall within the scope of the present invention. Subjects may be administered with the pharmaceutical composition at such doses daily, every other day, weekly, or according to any other regimen determined by empirical analysis. Exemplary dosage regimens include that the pharmaceutical composition is administered at 0.01-100 mg/kg for consecutive days. It is necessary to assess a therapeutic effect and the safety during administration with the drug of the present invention.

Product or kit

An embodiment of the present invention relates to a product or kit, which includes a component of a plasminogen activation pathway or its related compound (e.g., plasminogen). Preferably, the product includes a container with a label or package insert. Suitable containers include bottles, vials, syringes, etc. The container can be made from a variety of materials such as glass and plastic. The container contains a composition that can be used to treat the disease or symptoms of the present invention, and has a sterile inlet (e.g., the container may be an intravenous solution pack or vial with a plug that can be penetrated by a hypodermic needle). At least one active ingredient in the composition is a component of a plasminogen activation pathway or its related compound (e.g., plasminogen). The label attached to the container is used to describe that the composition is used to treat the symptoms of the present invention. The product may also include a second container containing a medicinal buffer such as phosphate-buffered saline, a Ringer's solution, and a dextrose solution. The product may also include other substances required from a commercial and user standpoint, which include other buffers, a diluent, a filter, a needle, and a syringe. In addition, the product includes a package insert with instructions for use, which are used to, for example, indicate a user of the composition to administrate the component of the plasminogen activation pathway or its related compound (e.g. plasminogen) and other drugs for treating concomitant diseases to a patient.

**Examples**

Plasminogen used in all the following examples was from plasma of a human donor, that is, was isolated from plasma of a human donor and purified by a method optimized with reference to the methods described in the following documents: Kenneth C Robbins, Louis Summaria, David Elwyn, et al. Further Studies on the Purification and Characterization of Human Plasminogen and Plasmin. Journal of Biological Chemistry, 1965, 240 (1): 541-550; Summaria L, Spitz F, Arzadon L, et al. Isolation and characterization of the affinity chromatography forms of human Glu- and Lys-plasminogens and plasmins. J Biol Chem. 1976 Jun 25; 251 (12): 3693-9; HAGAN JJ, ABLONDI FB, DE RENZO EC. Purification and biochemical properties of human plasminogen. J Biol Chem. 1960 Apr; 235: 1005-10. The purity of plasminogen monomer is greater than 98%.

**Example 1 Plasminogen improves a spontaneous activity and a phobotaxis behavior of Parkinson's model mice.**

Before one day of model construction, twenty-eight 10-12-week-old male C57BL/6J mice were weighed and were randomly divided into two groups according to body weights, i.e. a blank control group and a model group, 8 mice in the blank control group and 20 mice in the model group. Each mouse in the blank control group was intraperitoneally injected with 200 μl of normal saline solution. Each mouse in the model group was intraperitoneally injected with 1-methyl-4-phenyl-1,2,3,6-tetrahydropyridine (MPTP) solution for 5 consecutive days at a dose of 35 mg/kg to establish a Parkinson's model[1]. Preparation of MPTP solution: 45 mg of MPTP (Sigma, M0896) was taken and dissolved in 9 ml of normal saline solution to prepare a final concentration of 5 mg/ml. After the model construction was completed, on the 6th day after model construction, all mice were subjected to body weight measurement and open-field behavioral testing. The mice in the model group were randomly divided into two groups according to body weight and open field results, i.e., an administration group and a solvent group, 10 mice in the administration group and 10 mice in the solvent group. The day on which administration was started was denoted as the 1st day. The plasminogen was injected into each mouse of the administration group via the tail vein at a dose of 1 mg/100 μl, and a solvent solution (10 mM of citric acid-sodium citrate solution, pH 7.4) was injected into each mouse of the solvent group at a dose of 100 μl, and administration was performed for 14 consecutive days. An open field test was performed on the 15th day of administration.

MPTP is a specific strong substantia nigra toxin, and its metabolite MPP+ (1-methyl-4-phenylpyridinium) is an inhibitor of mitochondrial complex I, which can penetrate the blood-brain barrier, block the mitochondrial oxidative respiratory chain and other pathways from damaging dopamine neurons in the substantia nigra and striatum, and simulate human Parkinson's symptoms[2].

Open field test

In the test, the mouse was placed in the center of the bottom surface of the open field (40×40×40 cm) while video recording and timing were performed at the same time. The mouse was continuously observed for 5 min, and 3 tests were performed on each mouse. Recorded parameters include a total travel distance, a boundary resting time rate, a boundary zone travel distance, a percentage of boundary zone travel distance, a percentage of boundary zone slow travel time, a percentage of boundary zone time, the number of entries into the boundary zone, a central zone travel distance, a percentage of central zone travel distance, a maximum travel speed in the central zone, a percentage of central zone time, the number of entries into the central zone, and travel trails. In each test, a box was wiped with 70% ethanol to prevent the preference caused by odor.

The open field test is designed based on the phobotaxis of mice, which means that mice are afraid of open, unknown, and potentially dangerous places, and thus have a natural tendency to move "against the wall". A total distance and an average speed are regarded as main data reflecting spontaneous activities of a mouse, and the phobotaxis is assessed based on activities of the mouse in surrounding zones (four corners and four sides) of the open field. In view of activity time in the surrounding zones that reflects the phobotaxis, if the time is shorter, the mouse is more "adventurous". If the activity time in the central zone is longer, the phobotaxis and the anxiety (depression) level are lower.

**General activities**

**Total travel distance**

A total travel distance refers to the length of motion trails within the specified testing time.

The results show that each mouse in the blank control group has a certain travel distance during the test; a total travel distance of each mouse in a plasminogen administration group is significantly shorter than that in the solvent control group, and the statistical difference is significant (* means P<0.05); and a total travel distance of each mouse in the plasminogen administration group is close to that in the blank control group (see FIG. 1). It indicates that the plasminogen can promote the recovery of spontaneous activity behaviors of Parkinson's model mice.

**Activities in the boundary zone**

Resting time rate in the boundary zone

The boundary zone is surrounding zones (four corners and four sides) of the open field. The resting time rate in the boundary zone refers to a ratio of the resting time in the boundary zone to the total resting time (including the resting time in the boundary zone and the resting time in the central zone). The results show that each mouse in the blank control group has a certain resting time rate in the boundary zone; the resting time rate in the boundary zone of each mouse in the plasminogen administration group is significantly greater than that in the solvent control group, and the statistical difference is extremely significant (** indicates P<0.01); and the boundary resting time rate of each mouse in the plasminogen administration group is close to that in the blank control group (see FIG. 2). It indicates that the plasminogen can enhance the phobotaxis of Parkinson's model mice and relieve their anxiety.

Boundary zone travel distance

The boundary zone travel distance refers to the length of motion trails in the boundary zone within the specified testing time. The results show that each mouse in the blank control group has a certain boundary zone travel distance; the boundary zone travel distance of each mouse in the solvent control group is significantly longer than that in the plasminogen administration group, and the statistical difference is close to significant ($P=0.05$); and the boundary zone travel distance of each mouse in the plasminogen administration group is close to that in the blank control group (see FIG. 3). This indicates that the plasminogen can enhance the phobotaxis of Parkinson's model mice and relieve their anxiety.

Percentage of boundary zone travel distance

The percentage of boundary zone travel distance refers to a ratio of the boundary zone travel distance to the total travel distance (including the boundary zone travel distance and the central zone travel distance).

The results show that each mouse in the blank control group has a certain percentage of a boundary zone travel distance; the percentage of the boundary zone travel distance of each mouse in the plasminogen administration group is significantly greater than that in the solvent control group, and the statistical difference is extremely significant (** indicates $P<0.01$); and the percentage of the boundary zone travel distance of each mouse in the plasminogen administration group is close to that in the blank control group (see FIG. 4). It indicates that the plasminogen can enhance the phobotaxis of Parkinson's model mice and relieve their anxiety.

Percentage of boundary zone slow travel distance

The percentage of boundary zone slow travel time refers to a ratio of the boundary zone slow travel distance to the total testing time within the testing time.

The results show that each mouse in the blank control group has a certain percentage of boundary zone slow travel time; the percentage of the boundary zone slow travel time of each mouse in the plasminogen administration group is significantly less than that in the solvent control group, and the statistical difference is extremely significant (** indicates $P<0.01$); and the percentage of the boundary zone slow travel time of each mouse in the plasminogen administration group is close to that in the blank control group (see FIG. 5). This indicates that the plasminogen can enhance the phobotaxis of Parkinson's model mice and relieve their anxiety.

Percentage of boundary zone time

The percentage of boundary zone time refers to a ratio of the boundary zone time to the total testing time.

The results show that each mouse in the blank control group has a certain percentage of boundary zone time; the percentage of boundary zone time of each mouse in the plasminogen administration group is significantly greater than that in the solvent control group, and the statistical difference is close to significant ($P=0.06$); and the percentage of the boundary zone time of each mouse in the plasminogen administration group is close to that in the blank control group (see FIG. 6). This indicates that the plasminogen can enhance the phobotaxis behavior level of Parkinson's model mice and relieve their anxiety.

Number of entries into the boundary zone

The results show that each mouse in the blank control group has a certain number of entries into the boundary zone; the number of entries of each mouse in the plasminogen administration group into the boundary zone is significantly less than that in the solvent control group, and the statistical difference is significant (* indicates $P<0.05$); and the number of entries of each mouse in the plasminogen administration group into the boundary zone is close to that in the blank control group (see FIG. 7). This indicates that the plasminogen can enhance the phobotaxis of Parkinson's model mice and relieve their anxiety.

**Activities in the central zone**

Central zone travel distance

The central zone travel distance refers to the length of motion trails in the central zone within the testing time.

The results show that each mouse in the blank control group has a certain central zone travel distance; the central zone travel distance of each mouse in the plasminogen administration group is significantly shorter than that in the solvent control group, and the statistical difference is significant (* indicates $P<0.05$); and the central zone travel distance of each mouse in the plasminogen administration group is close to that in the blank control group (see FIG. 8). This indicates that the plasminogen can enhance the phobotaxis of Parkinson's model mice and relieve their anxiety.

Percentage of central zone travel distance

The percentage of central zone travel distance refers to a ratio of the length of motion trails in the central zone within the testing time and a total motion trail length.

The results show that each mouse in the blank control group has a certain percentage of a central zone travel distance; the percentage of a central zone travel distance of each mouse in the plasminogen administration group is significantly less than that in the solvent control group, and the statistical difference is significant (* indicates P<0.05); and the percentage of a central zone travel distance of each mouse in the plasminogen administration group is close to that in the blank control group (see FIG. 9). This indicates that the plasminogen can enhance the phobotaxis of Parkinson's model mice and relieve their anxiety.

Maximum central zone travel speed

The maximum central zone travel speed refers to the fastest travel speed of the central zone within the testing time.

The results show that each mouse in the blank control group has a certain maximum central zone travel speed; the maximum central zone travel speed of each mouse in the plasminogen administration group is significantly less than that in the solvent control group, and the statistical difference is extremely significant (** indicates P<0.01); and the maximum central zone travel speed of each mouse in the plasminogen administration group is close to that in the blank control group (see FIG. 10). This indicates that the plasminogen can enhance the phobotaxis of Parkinson's model mice and relieve their anxiety.

Percentage of central zone time

The percentage of central zone time refers to a ratio of the travel time of the central zone to the total testing time.

The results show that each mouse in the blank control group has a certain percentage of time of entering the central zone; the percentage of the central zone time of each mouse in the plasminogen administration group is significantly less than that in the solvent control group, and the statistical difference is close to significant (P=0.06); and the percentage of the central zone time of each mouse in the plasminogen administration group is close to that in the blank control group (see FIG. 11). This indicates that the plasminogen can enhance the phobotaxis of Parkinson's model mice and relieve their anxiety.

Number of entries into the central zone

The results show that each mouse in the blank control group scarcely enters the central zone, showing a normal phobotaxis; the number of entries of each mouse in the plasminogen administration group into the central zone is significantly less than that in the solvent control group, and the statistical difference is significant (* indicates P<0.05); and the number of entries of each mouse in the plasminogen administration group into the central zone is close to that in the blank control group (see FIG. 12). This indicates that the plasminogen can enhance the phobotaxis of Parkinson's model mice and relieve their anxiety.

**Motion trails**

The results show that each mouse in the blank control group during the open field test has a certain total travel distance and almost no activity in the central zone; and each mouse in the plasminogen administration group shows a trend of significantly reduced total travel distance and activities in the central zone compared with that in the solvent control group, which is close to that in the blank control group (FIG. 13). This indicates that the plasminogen can enhance the phobotaxis of Parkinson's model mice and relieve their anxiety.

**Example 2 Plasminogen can promote the expression of DTA in the substantia nigra of Parkinson's model mice.**

Before one day of model construction, forty 10-12-week-old male C57BL/6J mice were weighed and were randomly divided into two groups according to body weights, i.e. a blank control group and a model group, 8 mice in the blank control group and 32 mice in the model group. Each mouse in the blank control group was intraperitoneally injected with 200 $\mu$l of normal saline solution. Each mouse in the model group was intraperitoneally injected with 1-methyl-4-phenyl-1,2,3,6-tetrahydropyridine (MPTP) solution for 5 consecutive days at a dose of 40 mg/kg to establish a Parkinson's model[1]. Preparation of MPTP solution: 45 mg of MPTP (Sigma, M0896) was taken and dissolved in 9 ml of normal saline solution to prepare a final concentration of 5 mg/ml. After model construction, i.e., the 6th day of model construction, the mice in the model group were randomly divided into two groups according to body weights, i.e., a solvent group and an administration group, 16 mice in the administration group and 16 mice in the solvent group. The day on which administration was started was denoted as the 1st day. A plasminogen solution was injected into each mouse of the administration group via the tail vein at a dose of 1 mg/100 $\mu$l, and a solvent solution (10 mM of citric acid-sodium citrate solution, pH 7.4) was injected into each mouse in the solvent group at a dose of 100 $\mu$l, and administration was performed for 14 consecutive days. The mice were killed on the 15th day of administration, and the substantia nigra of the mice were harvested and fixed in a 10% neutral formaldehyde solution for 24-48 hours. The fixed substantia nigra tissue was dehydrated with graded ethanol, cleared with xylene, and embedded in paraffin. The slice has a thickness of 3 $\mu$m and subjected to deparaffinage and rehydration, and then washed once with water. The slice was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse dopamine transporter (DAT) antibody (ab7260, Abeam) was added dropwise, and the slice was incubated at 4°C overnight, and washed twice with 0.01 M PBS for 5 min each time. A goat anti-rabbit IgG (HRP) antibody (Abcam) secondary antibody was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

Parkinson disease (PD) is a neurodegenerative disease pathologically characterized by the progressive death of nigrostriatal dopaminergic neurons and the formation of Lewy bodies in the cytoplasm of residual dopaminergic neurons, as well as the deficiency of nodal dopamine, thereby leading to the development of classic dyskinesia symptoms of PD. A dopamine transporter (DAT) is located in the presynaptic membrane of dopamine neurons and can re-uptake dopamine transmitters released to the synaptic cleft, reflecting a presynaptic function of dopamine neurons. DAT reduction is closely related to the development of PD[3].

The results show that dopamine neurons in the substantia nigra of each mouse in the blank control group (see FIG. 14A) express a certain amount of DTA (marked by arrows), and the expression quantity of DTA in the substantia nigra of each mouse in the solvent group (see FIG. 14B) is lower than that in the blank control group, while the expression quantity of DTA in the substantia nigra of each mouse in the plasminogen administration group (see FIG. 14C) is higher than that in the solvent group. It results indicate that the plasminogen can promote the expression of DTA in the substantia nigra of Parkinson's model mice.

**Example 3 Plasminogen can promote the recovery of Nissl bodies of the striatum in Parkinson's model mice.**

Before one day of model construction, forty 10-12-week-old male C57BL/6J mice were weighed and were randomly divided into two groups according to body weights, i.e. a blank control group and a model group, 8 mice in the blank control group and 32 mice in the model group. Each mouse in the blank control group was intraperitoneally injected with 200 μl of normal saline solution. Each mouse in the model group was intraperitoneally injected with 1-methyl-4-phenyl-1,2,3,6-tetrahydropyridine (MPTP) solution for 5 consecutive days at a dose of 40 mg/kg to establish a Parkinson's model[1]. Preparation of MPTP solution: 45 mg of MPTP (Sigma, M0896) was taken and dissolved in 9 ml of normal saline solution to prepare a final concentration of 5 mg/ml. After model construction, i.e., the 6th day of model construction, the mice in the model group were randomly divided into two groups according to body weights, i.e., a solvent group and an administration group, 16 mice in the solvent group and 16 mice in the administration group. The day on which administration was started was denoted as the 1st day. A plasminogen solution was injected into each mouse in the administration group via the tail vein at a dose of 1 mg/100 μl, and a solvent solution (10 mM of citric acid-sodium citrate solution, pH 7.4) was injected into each mouse in the solvent group at a dose of 100 μl, and administration was performed for 14 consecutive days. The mice were killed on the 15th day of administration, and the substantia nigra of the mice was harvested and fixed in a 10% neutral formaldehyde solution for 24-48 hours. The fixed striatum tissue was dehydrated with graded ethanol, cleared with xylene, and embedded in paraffin. A tissue slice with a thickness of 3 μm was subjected to deparaffinage to water, and stained with a 0.4% tar violet staining solution (pH=3). The slice was dehydrated with graded ethanol, cleared with xylene, and sealed by a neutral gum. The slice was observed under a 400× optical microscope and photographed.

The main pathological change of PD is the progressive degeneration and necrosis of nigrostriatal dopaminergic neurons, which leads to a decrease in the level of striatal dopamine transmitters, thereby causing the dysfunction of the entire basal ganglia circuit[3]. Nissl body, also known as chromatin, is a unique structure of nerve cells. It consists of many parallel rough endoplasmic reticulums and free ribosomes during distribution, and has a function of synthesizing proteins. The number and distribution of the Nissl bodies are closely related to the functional state of neurons and are regarded as markers of neurocyte survival[4]. The results show that striatal neurons of each mouse in the blank control group (see FIG. 15A) have a certain number of Nissl bodies (marked by arrows); the number of Nissl bodies in striatal neurons of each mouse in the solvent control group (see FIG. 15B) is significantly more than that in the blank control group, and the statistical difference is close to significant (P=0.085); the number of Nissl bodies in striatal neurons of each mouse in the plasminogen administration group (see FIG. 15C) has no significant difference from that in the blank control group, but is significantly lower than that in the solvent control group, and the statistical difference is significant (* indicates P<0.05) (see FIG. 15D). The results indicate that the plasminogen can promote the recovery of Nissl bodies in the striatum of Parkinson's model mice.

**Example 4 Plasminogen can promote the recovery of the number of Nissl bodies in the substantia nigra of Parkinson's model mice.**

Before one day of model construction, twenty-eight 10-12-week-old male C57BL/6J mice were weighed and were randomly divided into two groups according to body weights, i.e. a blank control group and a model group, 8 mice in the blank control group and 20 mice in the model group. The model construction time was set at 9:00 a.m. every day. Each mouse in the blank control group was injected with 200 $\mu$l of normal saline; and each mouse in the model group was injected with a 5 mg/ml MPTP solution at a dose of 35 mg/kg for 5 consecutive days to establish a Parkinson's model[5]. Preparation of MPTP solution: 45 mg of MPTP (sigma, M0896) was taken and dissolved in 9 ml of normal saline solution to prepare a final concentration of 5 mg/ml. After model construction, i.e., the 6th day of model construction, the mice in the model group were randomly divided into two groups according to body weights, i.e., a solvent group and an administration group, 10 mice in the solvent group and 10 mice in the administration group. The day on which administration was started was denoted as the 1st day. A plasminogen solution was injected into each mouse in the administration group via the tail vein at a dose of 1 mg/100 $\mu$l, and a solvent solution (10 mM of citric acid-sodium citrate solution, pH 7.4) was injected into each mouse in the solvent group at a dose of 100 $\mu$l, and administration was performed for 14 consecutive days. The mice were killed on the 15th day of administration, and the substantia nigra of the mice was harvested and fixed in a 10% neutral formaldehyde solution for 24-48 hours. The fixed substantia nigra tissue was dehydrated with graded ethanol, cleared with xylene, and embedded in paraffin. A tissue slice with a thickness of 3 $\mu$m was subjected to deparaffinage to water, and stained with a 0.4% tar violet staining solution (pH=3). The slice was dehydrated with graded ethanol, cleared with xylene, and sealed by a neutral gum. The slice was observed under a 400$\times$ optical microscope and photographed.

The results show that each mouse in the blank control group (see FIG. 16A) has a certain amount of Niss1 bodies (marked by arrows) in the substantia nigra; the number of Nissl bodies in the substantia nigra of each mouse in the solvent control group (see FIG. 16B) decreases; and the number of Nissl bodies in the substantia nigra of each mouse in the plasminogen administration group (see FIG. 16C) is higher than that in the solvent group. The results indicate that the plasminogen can promote the recovery of the number of Nissl bodies in the substantia nigra of Parkinson's model mice.

**Example 5 Plasminogen can promote the expression of GLP-1R in the substantia nigra of Parkinson's model mice.**

Twelve 9-week-old C57 male mice were weighed 1 day before model construction. Each mouse was injected intraperitoneally with a 5 mg/ml MPTP solution at a dose of 30 mg/kg/body weight every day for 5 consecutive days to establish a Parkinson's model[6-7]. Preparation of MPTP solution: 10 ml of deionized water is sucked with a syringe, and added to 100 mg of MPTP powder (sigma, M0896) to prepare a 10 mg/ml mother solution; and 1 ml of the mother solution was sucked into an ampoule and then added with 1 ml of deionized water to a final concentration of 5 mg/ml. After model construction was completed, the mice were randomly divided into two groups, i.e., a solvent PBS control group and a plasminogen administration group, 6 mice in the solvent PBS control group and 6 mice in the plasminogen administration group. The day on which administration was started was denoted as the 1st day. A plasminogen solution was injected into each mouse of the plasminogen administration group via the tail vein at a dose of 1 mg/0.1 ml/day, and also injected into the solvent PBS control group via the tail vein at the same dose, and administration was performed for 14 consecutive days. The mice were killed on the 15th day of administration, and brains were rapidly harvested and fixed in 4% paraformaldehyde for 24-48 hours. The fixed brain tissue was dehydrated with graded ethanol, cleared with xylene, and embedded in paraffin. The substantia nigra on the slice was positioned, and the slice has a thickness of 4 $\mu$m and is subjected to deparaffinage and rehydration, and then washed once with water. The tissue was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse GLP-1R antibody (NOVUS, NBP1-97308) was added dropwise, and the slice was incubated at 4°C overnight, and washed twice with 0.01 M PBS for 5 min each time. A goat anti-rabbit IgG (HRP) antibody (Abcam) secondary antibody was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 200$\times$ optical microscope.

A glucagon-like peptide 1 receptor (GLP-1R), a member of the glucagon receptor family, is a G protein-coupled receptor that can regulate the blood sugar level by promoting insulin secretion[8-9]. Parkinson's disease is characterized by the loss of dopaminergic signaling in nigrostriatal neurons, which also express GLP-1R[10].

The results show that the expression quantity (marked by arrows) of GLP-1R in the substantia nigra of each mouse in the plasminogen administration group (see FIG. 17B) is significantly higher than that in the solvent PBS control group (see FIG. 17A), and the statistical difference is significant (* indicates P<0.05) (see FIG. 17C). The results indicate that the plasminogen can enhance the expression of GLP-1R in the substantia nigra of Parkinson's model mice.

**Example 6 Plasminogen can promote the expression of TH in the substantia nigra of Parkinson's model mice.**

Twelve 9-week-old C57 male mice were weighed 1 day before model construction. Each mouse was injected intraperitoneally with a 5 mg/ml MPTP solution at a dose of 30 mg/kg/body weight every day for 5 consecutive days to establish a Parkinson's model[6-7]. Preparation of MPTP solution: 10 ml of deionized water is sucked with a syringe, and added to 100 mg of MPTP powder (sigma, M0896) to prepare a 10 mg/ml mother solution; and 1 ml of the mother solution was sucked into an ampoule and then added with 1 ml of deionized water to a final concentration of 5 mg/ml. After model construction was completed, the mice were randomly divided into two groups, i.e., a solvent PBS control group and a plasminogen administration group, 6 mice in the solvent PBS control group and 6 mice in the plasminogen administration group. The day on which administration was started was denoted as the 1st day. A plasminogen solution was injected into each mouse of the plasminogen administration group via the tail vein at a dose of 1 mg/0.1 ml/day, and also injected into the solvent PBS control group via the tail vein at the same dose, and administration was performed for 14 consecutive days. The mice were killed on the 15th day of administration, and substantia nigra of each mouse was rapidly harvested and fixed in 4% paraformaldehyde for 24-48 hours. The fixed brain tissue was dehydrated with graded ethanol, cleared with xylene, and embedded in paraffin. The substantia nigra on the slice was positioned, and the slice has a thickness of 3 μm and is subjected to deparaffinage and rehydration, and then washed once with water. The tissue was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse TH antibody (Proteintech, 25859-1-AP) was added dropwise, and the slice was incubated at 4°C overnight, and washed twice with 0.01 M PBS for 5 min each time. A goat anti-rabbit IgG (HRP) antibody (Abcam) secondary antibody was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

Tyrosine hydroxylase (TH) is a rate-limiting enzyme for tyrosine synthesis of L-dopa, and is only expressed in the cytoplasm and is abundant in dopamine neurons. Most of the TH-positive neurons in the substantia nigra are dopaminergic, so TH can be used as a marker of dopaminergic neurons in the substantia nigra, and the expression quantity of TH in the substantia nigra becomes an indicator for detecting PD[11].

The results show that each mouse in the blank control group (see FIG. 18A) has a certain amount of TH-positive cells (marked by arrows) in the substantia nigra; the number of TH-positive cells in the substantia nigra of each mouse in the solvent group (see FIG. 18B) decreases; and the number of TH-positive cells in the substantia nigra of each mouse in the plasminogen administration group (see FIG. 18C) is significantly higher than that of each mouse in the solvent group. The results indicate that the plasminogen can increase the number of TH-positive cells in the substantia nigra of Parkinson's model mice.

**Example 7 Plasminogen can affect the number of microglia in the substantia nigra of Parkinson's model mice.**

Twelve 9-week-old C57 male mice were weighed 1 day before model construction. Each mouse was injected intraperitoneally with a 5 mg/ml MPTP solution at a dose of 30 mg/kg/body weight every day for 5 consecutive days to establish a Parkinson's model[6-7]. Preparation of MPTP solution: 10 ml of deionized water is sucked with a syringe, and added to 100 mg of MPTP powder (sigma, M0896) to prepare a 10 mg/ml mother solution; and 1 ml of the mother solution was sucked into an ampoule and then added with 1 ml of deionized water to a final concentration of 5 mg/ml. After model construction was completed, the mice were randomly divided into two groups, i.e., a solvent PBS control group and a plasminogen administration group, 6 mice in the solvent PBS control group and 6 mice in the plasminogen administration group. The day on which administration was started was denoted as the 1st day. A plasminogen solution was injected into each mouse of the plasminogen administration group via the tail vein at a dose of 1 mg/0.1 ml/day, and also injected into the solvent PBS control group via the tail vein at the same dose, and administration was performed for 14 consecutive days. The mice were killed on the 15th day of administration, and substantia nigra of each mouse was harvested and fixed in 4% paraformaldehyde for 24-48 hours. The fixed substantia nigra was dehydrated with graded ethanol, cleared with xylene, and embedded in paraffin. The substantia nigra on the slice was positioned, and the slice has a thickness of 3 μm and is subjected to deparaffinage and rehydration, and then washed once with water. The tissue was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse Iba-1 antibody (Abcam, ab178847) was added dropwise, and the slice was incubated at 4°C overnight, and washed twice with 0.01 M PBS for 5 min each time. A goat anti-rabbit IgG (HRP) antibody (Abcam) secondary antibody was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

Microglia are innate immune cells of the central nervous system that are activated upon brain lesions or injury. Activated microglia migrate to an injury site and exert various functions, such as phagocytosis of dead cells, increase of pro-inflammatory cytokines, etc., and are involved in various central nervous system diseases[12-14]. Iba-1 (Ionized calcium binding adapter molecule 1) is a calcium-binding protein of about 17kDa, which is specifically expressed in the central nervous system microglia and has been widely used as a microglia marker[15-17].

The results show that each mouse in the blank control group (see FIG. 19A) has a certain amount of microglia (marked by arrows) in the substantia nigra; the number of microglia in the substantia nigra of each mouse in the solvent control group (see FIG. 19B) is greater than that of each mouse in the blank control group; and the number of microglia in the substantia nigra of each mouse in the plasminogen administration group (see FIG. 19C) is significantly less than that in the solvent control group, and close to that in the blank control group. The results show that the plasminogen can affect the number of microglia in the substantia nigra of Parkinson's model mice.

**Example 8 Plasminogen can promote the recovery of myelin sheaths of the striatum in Parkinson's model mice.**

Twelve 9-week-old C57 male mice were weighed 1 day before model construction. Each mouse was injected intraperitoneally with a 5 mg/ml MPTP solution at a dose of 30 mg/kg/body weight every day for 5 consecutive days to establish a Parkinson's model[6-7]. Preparation of MPTP solution: 10 ml of deionized water is sucked with a syringe, and added to 100 mg of MPTP powder (sigma, M0896) to prepare a 10 mg/ml mother solution; and 1 ml of the mother solution was sucked into an ampoule and then added with 1 ml of deionized water to a final concentration of 5 mg/ml. After model construction was completed, the mice were randomly divided into two groups, i.e., a solvent PBS control group and a plasminogen administration group, 6 mice in the solvent PBS control group and 6 mice in the plasminogen administration group. The day on which administration was started was denoted as the 1st day. A plasminogen solution was injected into each mouse in the plasminogen administration group via the tail vein at a dose of 1 mg/0.1 ml/day, and PBS was injected into each mouse in the solvent PBS control group via the tail vein at the same volume, and administration was performed for 14 consecutive days. The mice were killed on the 15th day of administration, and striatum of each mouse was harvested and fixed in a 10% normal formaldehyde solution for 24-48 hours. The fixed striatum tissue was dehydrated with graded ethanol, cleared with xylene, and embedded in paraffin. A tissue slice with a thickness of 3 μm was subjected to deparaffinage to water, and stained by sealed impregnation with a 0.1% LFB staining solution for 8-16h. The slice was dehydrated with graded ethanol, cleared with xylene, and sealed by a neutral gum. The slice was observed under a 400× optical microscope and photographed.

Neurodegenerative diseases refer to diseases caused by the loss of neurons or their myelin sheaths in the brain and spinal cord. LFB (Luxol fast blue) is a myelin sheath-specific staining method that can reflect the damage of myelin sheaths[18-19].

The results show that each mouse in the blank control group (see FIG. 20A) has a certain amount of myelin sheaths in the striatum; the staining of myelin sheaths in the striatum of each mouse in the solvent group (see FIG. 20B) is less than that in the blank control group; and the staining of myelin sheaths in the striatum of each mouse in the plasminogen administration group (see FIG. 20C) is significantly greater than that in the solvent group, and close to that in the blank control group. The results indicate that the plasminogen can promote the recovery of myelin sheaths in the striatum of Parkinson's model mice to a certain degree.

**Example 9 Plasminogen can promote the expression of α-synuclein in the substantia nigra of Parkinson's model mice.**

Before one day of model construction, twenty-eight 10-12-week-old male C57BL/6J mice were weighed and were randomly divided into two groups according to body weights, i.e. a blank control group and a model group, 8 mice in the blank control group and 20 mice in the model group. The model construction time was set at 9:00 a.m. every day. Each mouse in the blank control group was injected with 200 μl of normal saline; and each mouse in the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After model construction, i.e., the 6th day of model construction, the mice in the model group were randomly divided into two groups according to body weights, i.e., a solvent group and an administration group, 10 mice in the solvent group and 10 mice in the administration group. The day on which administration was started was denoted as the 1st day. A plasminogen solution was injected into each mouse in the administration group via the tail vein at a dose of 1 mg/100 μl, and a solvent so-

lution (10 mM of citric acid-sodium citrate solution, pH 7.4) was injected into each mouse of the solvent group at a dose of 100 μl, and administration was performed for 14 consecutive days. The mice were killed on the 15th day of administration, and the substantia nigra of each mouse was harvested and fixed in 4% paraformaldehyde solution for 24-48 hours. The fixed brain tissue was dehydrated with graded ethanol, cleared with xylene, and embedded in paraffin. The substantia nigra on the slice was positioned, and the slice has a thickness of 3 μm and is subjected to deparaffinage and rehydration, and then washed once with water. The tissue was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse α-synuclein antibody (Proteintech, 10842-1-AP) was added dropwise, and the slice was incubated at 4°C overnight, and washed twice with 0.01 M PBS for 5 min each time. A goat anti-rabbit IgG (HRP) antibody (Abcam) secondary antibody was incubated at the room temperature for 1 h, and washed twice with 0.01 M PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

Parkinson's disease is currently regarded to be caused by the deficiency of dopaminergic neurons in the substantia nigra and the appearance of Lewy bodies. α-synuclein is a neuronal protein composed of 140 amino acid residues, which can cause neuronal damage and participate in the process of neurodegeneration in the central nervous system. Studies have shown that Lewy bodies of neurocyte and α-synuclein aggregated within synapses are markers of brain lesions in Parkinson's disease[20].

The results show that each mouse in the blank control group (see FIG. 21A) only has a small amount of α-synuclein in the substantia nigra; the amount of α-synuclein in the substantia nigra of the solvent group (see FIG. 21B) is significantly higher than that in the blank control group (* indicates P<0.05); the amount of α-synuclein in the substantia nigra of each mouse in the plasminogen administration group (see FIG. 21C) is significantly less than that in the solvent group and close to that in the blank control group, and the statistical difference is significant (* indicates P<0.05) (see FIG. 21D). This indicates that plasminogen can reduce the expression of α-synuclein in the substantia nigra of Parkinson's model mice and improve the neuron damage degeneration.

**Example 10 Plasminogen can improve the axonal injury of the striatum in Parkinson's model mice.**

Before one day of model construction, twenty-eight 10-12-week-old male C57BL/6J mice were weighed and were randomly divided into two groups according to body weights, i.e. a blank control group and a model group, 8 mice in the blank control group and 20 mice in the model group. The model construction time was set at 9:00 a.m. every day. The blank control group was injected with 200 μl of normal saline; and the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After model construction, i.e., the 6th day of model construction, the mice in the model group were randomly divided into two groups according to body weights, i.e., a solvent group and an administration group, 10 mice in the solvent group and 10 mice in the administration group. The day on which administration was started was denoted as the 1st day. A plasminogen solution was injected into each mouse in the administration group via the tail vein at a dose of 1 mg/100 μl, and a solvent solution (10 mM of citric acid-sodium citrate solution, pH 7.4) was injected into each mouse of the solvent group at a dose of 100 μl, and administration was performed for 14 consecutive days. The mice were killed on the 15th day of administration, and the substantia nigra of each mouse was harvested and fixed in 4% paraformaldehyde solution for 24-48 hours. The fixed brain tissue was dehydrated with graded ethanol, cleared with xylene, and embedded in paraffin. The substantia nigra on the slice was positioned, and the slice has a thickness of 3 μm and is subjected to deparaffinage and rehydration, and then washed once with water. The tissue was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse NF antibody (Abcam, ab207176) was added dropwise, and the slice was incubated at 4°C overnight, and washed twice with 0.01 M PBS for 5 min each time. A goat anti-rabbit IgG (HRP) antibody (Abcam, ab6721) secondary antibody was incubated at the room temperature for 1 h, and washed twice with 0.01 M of PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

There is axonal injury in the disease process of Parkinson's patients, and the severity of axonal injury may continue to increase with the prolongation of the disease course. Neurofilaments (NF) are the main skeletal protein of neural axons, which play a crucial role in maintaining normal neuronal morphology and axonal transport, and can be used as relevant biomarkers for axonal damage[21].

The results show that each mouse in the blank control group (see FIG. 22A) has a certain amount of NF (marked by arrows) in the striatum; the amount of NF in the striatum of each mouse in the solvent group (see FIG. 22B) is less than that in the blank control group; and the amount of NF in the striatum of each mouse in the plasminogen administration group (see FIG. 22C) is significantly greater than that in the solvent group, and the statistical difference is extremely significant (** indicates P<0.01) (see FIG. 22D). The results indicate that the plasminogen can promote the recovery of NF expression of the striatum in Parkinson's model mice, and improve Parkinson's axonal damage.

**Example 11 Plasminogen can promote the expression of GFAP in the striatum of Parkinson's model mice.**

Before one day of model construction, twenty-eight 10-12-week-old male C57BL/6J mice were weighed and were randomly divided into two groups according to body weights, i.e. a blank control group and a model group, 8 mice in the blank control group and 20 mice in the model group. The model construction time was set at 9:00 a.m. every day. Each mouse in the blank control group was injected with 200 $\mu$l of normal saline; and each mouse in the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After model construction, i.e., the 6th day of model construction, the mice in the model group were randomly divided into two groups according to body weights, i.e., a solvent group and an administration group, 10 mice in the solvent group and 10 mice in the administration group. The day on which administration was started was denoted as the 1st day. A plasminogen solution was injected into each mouse in the administration group via the tail vein at a dose of 1 mg/100 $\mu$l, and a solvent solution (10 mM of citric acid-sodium citrate solution, pH 7.4) was injected into each mouse of the solvent group at a dose of 100 $\mu$l, and administration was performed for 14 consecutive days. The mice were killed on the 15th day of administration, and the substantia nigra of each mouse was harvested and fixed in 4% paraformaldehyde solution for 24-48 hours. The fixed brain tissue was dehydrated with graded ethanol, cleared with xylene, and embedded in paraffin. The substantia nigra on the slice was positioned, and the slice has a thickness of 3 $\mu$m and is subjected to deparaffinage and rehydration, and then washed once with water. The tissue was marked by using a PAP pen, incubated in 3% hydrogen peroxide for 15 min, and washed twice with 0.01 M PBS for 5 min each time. The slice was blocked with a 5% normal goat serum (Vector laboratories, Inc., USA) for 30 min; and then, the goat serum was removed, a rabbit anti-mouse GFAP antibody (Abeam, ab7260) was added dropwise, and the slice was incubated at 4°C overnight, and washed twice with 0.01 M PBS for 5 min each time. A goat anti-rabbit IgG (HRP) antibody (Abcam, ab6721) secondary antibody was incubated at the room temperature for 1 h, and washed twice with 0.01 M of PBS for 5 min each time. The slice was developed by using a DAB kit (Vector laboratories, Inc., USA), washed three times with water, and re-stained with hematoxylin for 30 s, and washed with running water for 5 min. The slice was dehydrated with graded ethanol, cleared with xylene, and sealed by a neutral gum. The slice was observed under a 400× optical microscope.

Glial fibrillary acidic protein (GFAP) is an important component that constitutes astrocyte cell body collagen, and it exists only in astrocyte glial fibril intermediate filaments. It is a characteristic marker of cytoplasmic activation[22], has inflammatory damage to neurons, and degenerates neurons[21], thereby causing the occurrence of Parkinson's disease.

The results show that each mouse in the blank control group (see FIG. 23A) has a small amount of GFAP expressions (marked by arrows) in the striatum; the expression quantity of GFAP in the striatum of each mouse in the solvent group (see FIG. 23B) is significantly higher than that in the blank control group; and the expression quantity of GFAP in the striatum of each mouse in the plasminogen administration group (see FIG. 23C) is lower than that in the solvent group. This indicate that the plasminogen can reduce the GFAP expression in the striatum of Parkinson's model mice, and alleviate the damage of striatal neurons.

**Example 12 Plasminogen can promote the degradation of $\alpha$-synuclein in the cerebral homogenate of Parkinson's model mice.**

Before one day of model construction, eight 11-12-week-old male, 18-25 g C57BL/6J mice were weighed and were randomly divided into two groups according to body weights, i.e. a blank control group and a model group, 4 mice in the blank control group and 4 mice in the model group. The model construction time was set at 9:00 a.m. every day. Each mouse in the blank control group was injected with 200 $\mu$l of normal saline; and each mouse in the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After the model construction was completed, on the 6th day after model construction, all mice were subjected to open field test to identify that the model construction was successful. All mice were killed, the whole brain was taken out and weighed, 1× PBS (Thermo Fisher, pH=7.4, 10010-031) was added at a rate of 150 mg of tissue per 1 mL of PBS, the tissue was homogenized at 4°C (3-4 times, 1 min/time) and then centrifuged at 4°C (at 12000 rpm for 20 min), and a supernatant was taken and placed in a new EP tube.

Eppendorf (EP) tubes were divided into ① a blank control group, ② a solvent control group, and ③ a plasminogen group, and 5 parallels were set for each group. 21.5 μL of normal saline, 4.6 μL of plasminogen solution (2 mg/mL), 23.9 μL of cerebral homogenate of the mouse were added in each tube of the blank control group. 21.5 μL of α-synuclein solution (Shanghai Qiangyao Biotechnology Co., Ltd., customized expressed human α-synuclein, UniProtKB - P37840, 1.0 mg/mL), 4.6 μL of solvent solution (10 mM sodium citrate, 2% arginine hydrochloride, 3% mannitol, pH 7.4), and 23.9 μL of cerebral homogenate of the mouse were added in each tube of the solvent control group. 21.5 mL of α-synuclein solution (1.0 mg/mL), 4.6 μL of plasminogen solution (2 mg/mL), and 23.9 μL of cerebral homogenate of the mouse were added in each tube of the plasminogen group. After samples of respective groups are added, they were incubated at 37°C for 6 h, and 50 μL of 0.1% trifluoroacetic acid solution was added to terminate the reaction.

A 12% gel was prepared according to gel preparation instructions of a Tris-Tricine-SDS-PAGE gel preparation kit (Solarbio, P1320). A sample of each group was uniformly mixed with a 4× loading buffer (TaKaRa, e2139) in a volume ratio of 3: 1, the mixture was heated at 100°C for 5 min, cooled, and centrifuged for 2 min, and 20 μL of sample was loaded. Electrophoresis was performed at 30 V for 1.5 h and then at 100 V to the bottom of the gel. After electrophoresis was completed, the gel was peeled off, stained with a 1%₀ Coomassie brilliant blue staining solution (1 g of coomassie brilliant blue R250 was dissolved in 1000 mL of mixture of ethanol, acetic acid, and purified water in a volume ratio of 5: 2: 13) for 30 min, and destained with a destaining solution (a mixture of purified water, acetic acid, and anhydrous ethanol in a volume ratio of 17: 2: 1) to clear. The gel was photographed by using a biomolecular imager and subjected to quantitative scanning analysis.

The results show that in the cerebral homogenate of Parkinson's model mice, the amount of α-synuclein in the plasminogen group is significantly lower than that in the solvent control group (*** represents P<0.001), and the amount of each of polymers a and b is significantly lower than that in the solvent control group, and the difference is extremely significant (** represents P<0.01, *** represents P<0.001); and in the cerebral homogenate of normal mice, the amount of α-synuclein in the plasminogen group is significantly lower than that in the solvent control group, and the difference is extremely significant (*** represents P<0.001), and the amount of each of the polymers a and b is significantly lower than that in the solvent control group, and the difference is significant (* represents P<0.05, and ** represents P<0.01) (see FIG. 24). This indicates that the plasminogen can effectively degrade human α-synuclein and its polymers in the cerebral homogenates of Parkinson's model mice and normal mice.

**Example 13 Plasminogen can promote the degradation of α-synuclein in the cerebral homogenate of Parkinson's model mice.**

Before one day of model construction, eight 11-12-week-old male, 18-25 g C57BL/6J mice were weighed and were randomly divided into two groups according to body weights, i.e. a blank control group and a model group, 4 mice in the blank control group and 4 mice in the model group. The model construction time was set at 9:00 a.m. every day. The blank control group was injected with 200 μl of normal saline; and the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After the model construction was completed, on the 6th day after model construction, all mice were subjected to open field test to identify that the model construction was successful. All mice were killed, the whole brain was taken out and weighed, 1× PBS (Thermo Fisher, pH=7.4, 10010-031) was added at a rate of 150 mg of tissue per 1 mL of PBS, the tissue was homogenized at 4°C (3-4 times, 1 min/time) and then centrifuged at 4°C (at 12000 rpm for 20 min), and a supernatant was taken and placed in a new EP tube.

Eppendorf (EP) tubes were divided into ① a blank group, ② a blank control group, ③ a solvent control group, and ④ an administration group, and 5 parallels were set for each group. 21.5 μL of normal saline, 4.6 μL of solvent solution (including 10 mM sodium citrate, 2% arginine hydrochloride, and 3% mannitol, pH=7.4), and 23.9 μL of cerebral homogenate of the mouse were placed in each tube of the blank group. 21.5 μL of normal saline, 4.6 μL of plasminogen solution (2 mg/mL), and 23.9 μL of cerebral homogenate of the mouse were placed in each tube of the blank control group. 21.5 μL of α-synuclein solution (Shanghai Qiangyao Biotechnology Co., Ltd., customized expressed human α-synuclein, UniProtKB - P37840, 1.0 mg/mL), 4.6 μL of solvent solution, and 23.9 μL of cerebral homogenate of the mouse were placed in each tube of the solvent control group. 21.5 mL of α-synuclein solution (1.0 mg/mL), 4.6 μL of plasminogen solution (2 mg/mL), and 23.9 μL of cerebral homogenate of the mouse were placed in each tube of the plasminogen group. After samples of respective groups are added, they were incubated at 37°C for 6 h, and 50 μL of 0.1% trifluoroacetic acid solution was added to terminate the reaction.

A 12% gel was prepared according to gel preparation instructions of a Tris-Tricine-SDS-PAGE gel preparation kit (Solarbio, P1320). A sample of each group was uniformly mixed with a $4\times$ loading buffer (TaKaRa, e2139) in a volume ratio of 3: 1, the mixture was heated at 100°C for 5 min, cooled, and centrifuged for 2 min, and 20 $\mu$L of sample was loaded. Electrophoresis was performed at 30 V for 1.5 h and then at 100 V to the bottom of the gel. After electrophoresis was completed, the gel was peeled off and transferred to a PVDF membrane (GE, A29433753), and electrophoresis was performed at 15 V for 2 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human $\alpha$-synuclein antibody (Proteintech, 10842-1-AP) was added, the PVDF membrane was incubated at the room temperature for 3 h, and washed four times with TBST, a goat anti-rabbit IgG (HRP) antibody (Abcam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and quantitative analysis was performed by using Image J.

The results show that in the cerebral homogenate of Parkinson's model mice, the amount of $\alpha$-synuclein in the plasminogen group is significantly lower than that in the solvent control group, and the difference is extremely significant (** represents P<0.01); the amount of its polymer is significantly lower than that in the solvent control group, and the difference is extremely significant (***represents P<0.001); and in the cerebral homogenate of normal mice, the amount of $\alpha$-synuclein in the plasminogen group is significantly lower than that in the solvent control group, and the difference is extremely significant (** represents P<0.01); and the amount of its polymer is significantly lower than that in the solvent control group, and the difference is significant (*** represents P<0.001) (see FIG. 25). This indicates that the plasminogen can effectively degrade human $\alpha$-synuclein and its polymers in the cerebral homogenates of Parkinson's model mice and normal mice.

**Example 14 Plasminogen can promote the cleavage of Pro-BDNF in the cerebral homogenate of Parkinson's model mice.**

Four 11-12-week-old male, 18-25 g C57BL/6J mice were taken. The model construction time was set at 9:00 a.m. every day. The blank control group was injected with 200 $\mu$l of normal saline; and the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After the model construction was completed, on the 6th day after model construction, all mice were subjected to open field test to identify that the model construction was successful. After all mice were killed, the whole brain was taken out and weighed, $1\times$ PBS (Thermo Fisher, pH=7.4, 10010-031) was added at a rate of 150 mg of tissue per 1 mL of PBS, the tissue was homogenized at 4°C (3-4 times, 1 min/time) and then centrifuged at 4°C (at 12000 rpm for 20 min), and a supernatant, i.e. a cerebral homogenate was placed in a new EP tube.

Eppendorf (EP) tubes were divided into ① a blank group, ② a blank control group, ③ a solvent control group, and ④ a plasminogen group, and 5 parallels were set for each group. 21.5 $\mu$L of normal saline, 4.6 $\mu$L of solvent solution (including 10 mM sodium citrate, 2% arginine hydrochloride, and 3% mannitol, pH=7.4), and 23.9 $\mu$L of cerebral homogenate of the mouse were placed in each tube of the blank group. 21.5 $\mu$L of normal saline, 4.6 $\mu$L of plasminogen solution (2 mg/mL), and 23.9 $\mu$L of cerebral homogenate of the mouse were placed in each tube of the blank control group. 21.5 $\mu$L of Pro-BDNF (Nanjing GenScript, customized expressed human Pro-BDNF, UniProtKB - P23560, 1.0 mg/mL), 4.6 $\mu$L of solvent solution, and 23.9 $\mu$L of cerebral homogenate of the mouse were placed in each tube of the solvent control group. 21 $\mu$L of Pro-BDNF (1.0 mg/mL), 4.6 $\mu$L of plasminogen solution (2 mg/mL), and 23.9 $\mu$L of cerebral homogenate of the mouse were placed in each tube of the plasminogen group. After samples of respective groups are added, they were incubated at 37°C for 6 h, and 50 $\mu$L of 0.1 % trifluoroacetic acid solution was added to terminate the reaction.

A 12% gel was prepared according to gel preparation instructions of SDS-PAGE. A sample of each group was uniformly mixed with a $4\times$ loading buffer (TaKaRa, e2139) in a volume ratio of 3: 1, the mixture was heated at 100°C for 5 min, cooled, and centrifuged for 2 min, and 20 $\mu$L of sample was loaded. Electrophoresis was performed at 30 V for 45 min and then at 100 V to the bottom of the gel. After electrophoresis was completed, the gel was peeled off, stained with a 1‰ Coomassie brilliant blue staining solution (1 g of coomassie brilliant blue R250 was dissolved in 1000 mL of mixture of ethanol, acetic acid, and purified water in a volume ratio of 5: 2: 13) for 30 min, and destained with a destaining solution (a mixture of purified water, acetic acid, and anhydrous ethanol in a volume ratio of 17: 2: 1) to clear. The gel was photographed by using a biomolecular imager and subjected to quantitative scanning analysis.

Brain-derived neurotrophic factor (BDNF) is an alkaline protein having a molecular weight of 12.3 kDa, is composed of 119 amino acid residues, and contains three pairs of disulfide bonds. BDNF is present in the body in the form of dimer and synthesized in the form of a BDNF precursor (Pro-BDNF) that can be cleaved by enzymolysis to form mature BDNF. It has been reported in documents that Pro-BDNF has opposite effects to mature BDNF formed by cleaving Pro-BDNF. Pro-BDNF promotes apoptosis of nerve cells and reduces neural synaptic plasticity. Mature BDNF and its receptors are widely found in the central nervous system, and play an important role in the survival, differentiation, and growth and development of neurons during the development of the central nervous system. Furthermore, they can prevent neuronal damage and apoptosis, improve the pathological state of neurons, promote biological effects, such as regeneration and differentiation, of injured neurons, and are also necessary for the survival and normal physiological functions of neurons in the mature central and peripheral nervous systems[24].

The results show that in the cerebral homogenate of Parkinson's model mice, the amount of Pro-BDNF in the plasminogen group is significantly lower than that in the solvent control group, and the difference is extremely significant (*** represents $P<0.001$) (see FIG. 26). It indicates that the plasminogen can promote the cleavage of Pro-BDNF in the cerebral homogenate of Parkinson's model mice.

**Example 15 Plasminogen can promote the cleavage of Pro-BDNF in the cerebral homogenate of Parkinson's model mice into mature BDNF.**

Before one day of model construction, eight 11-12-week-old male, 18-25 g C57BL/6J mice were weighed and were randomly divided into two groups according to body weights, i.e. a blank control group and a model group, 4 mice in the blank control group and 4 mice in the model group. The model construction time was set at 9:00 a.m. every day. The blank control group was injected with 200 $\mu$l of normal saline; and the model group was intraperitoneally injected with a 5 mg/mL MPTP solution at a dose of 35 mg/kg for 5 consecutive days to establish a Parkinson's model[5]. Preparation of MPTP solution: 45 mg of MPTP (sigma, M0896) was taken and dissolved in 9 mL of normal saline solution to prepare a final concentration of 5 mg/mL. After the model construction was completed, on the 6th day after model construction, all mice were subjected to open field test to identify that the model construction was successful. After all mice were killed, the whole brain was taken out and weighed, $1\times$ PBS (Thermo Fisher, pH=7.4, 10010-031) was added at a rate of 150 mg of tissue per 1 mL of PBS, the tissue was homogenized at 4°C (3-4 times, 1 min/time) and then centrifuged at 4°C (at 12000 rpm for 20 min), and a supernatant, i.e. a cerebral homogenate was placed in a new EP tube.

Eppendorf (EP) tubes were divided into ① a blank group, ② a blank control group, ③ a solvent control group, and ④ a plasminogen group, and 5 parallels were set for each group. 21.5 $\mu$L of normal saline, 4.6 $\mu$L of plasminogen solution (2 mg/mL), and 23.9 $\mu$L of cerebral homogenate of the mouse were placed in each tube of the blank control group. 21.5 $\mu$L of Pro-BDNF (Nanjing GenScript, customized expressed human Pro-BDNF, UniProtKB - P23560, 1.0 mg/mL), 4.6 $\mu$L of solvent solution (citric acid-sodium citrate solution), and 23.9 $\mu$L of cerebral homogenate of the mouse were placed in each tube of the solvent control group. 21 $\mu$L of Pro-BDNF (1.0 mg/mL), 4.6 $\mu$L of plasminogen solution (2 mg/mL), and 23.9 $\mu$L of cerebral homogenate of the mouse were placed in each tube of the plasminogen group. After samples of respective groups are added, they were incubated at 37°C for 6 h, and 50 $\mu$L of 0.1% trifluoroacetic acid solution was added to terminate the reaction.

A 12% gel was prepared according to gel preparation instructions of SDS-PAGE. A sample of each group was uniformly mixed with a $4\times$ loading buffer (TaKaRa, e2139) in a volume ratio of 3: 1, the mixture was heated at 100°C for 5 min, cooled, and centrifuged for 2 min, and 20 $\mu$L of sample was loaded. Electrophoresis was performed at 30 V for 45 min and then at 100 V to the bottom of the gel. After electrophoresis was completed, the gel was peeled off and transferred to an activated PVDF membrane (GE, A29433753), and electrophoresis was performed at 15 V for 2.5 h. The transferred PVDF membrane was immersed in a blocking solution (a 5% skim emulsion) and blocked in a refrigerator at 4°C overnight, washed four times with TBST (a 0.01 M Tris-NaCl buffer, pH=7.6), a rabbit anti-human BDNF antibody (Boster Biological Technology, PB9075) was added, the PVDF membrane was incubated at the room temperature for 3 h, and washed four times with TBST, a goat anti-rabbit IgG (HRP) antibody (Abcam, ab6721) secondary antibody was added, the PVDF membrane was incubated at the room temperature for 1 h, washed four times with TBST, placed on a clean imaging plate, developed with Immobilon Western HRP Substrate (MILLIPORE, WBKLS0100), and photographed by using a biomolecular imager, and quantitative analysis was performed by using Image J.

The results show that in the cerebral homogenate of Parkinson's model mice, the amount of Pro-BDNF in the plasminogen group is significantly lower than that in the solvent control group, and the difference is significant (* represents $P<0.05$,*** represents $P<0.001$); and the amount of BDNF in the plasminogen group is significantly higher than that in the solvent control group, and the difference is extremely significant (see FIG. 27). It indicates that the plasminogen can promote the cleavage of Pro-BDNF in the cerebral homogenate of Parkinson's model mice and the formation of mature BDNF.

References

[1] M. Sedelis et al. Behavioral phenotyping of the MPTP mouse model of Parkinson's Disease[J].Behaioural Brain Research 125 (2001) 109-122.

[2] Meredith G E,Totterdell S,Potashkin J A,et al. Modeling PD pathogenesis in mice advantages of a chronic MPTP protocol[J]. Parkinsonism Relat Disord,2008,14(Suppl 2):S112-S115.

[3] Ken Ikeda, Junya Ebina, Kiyokazu Kawabe and Yasuo Iwasaki. Dopamine Transporter Imaging in Parkinson Disease:Progressive Changes and Therapeutic Modification after Anti-parkinsonian Medications [J].Internal Medicine,2019, 58: 1665-1672.

[4] HEBERT AE, DASH PK. Nonredundant roles for hippocampal and entorhinal cortical plasticity in spatial memory storage [J]. Pharmacology Biochemistry and Behavior, 2004, 79(1) :143-153.

[5] Won-Seok Choi, et al. Conditional deletion of Ndufs4 in dopaminergic neurons promotes Parkinson's disease-like nonmotor symptoms without loss of dopamine neurons. Scientific Reports.

[6] Vernice Jackson-Lewis1 & Serge Przedborski. Protocol for the MPTP mouse model of Parkinson's Disease [J]. Nature protocols VOL.2 NO.1, 2007(141).

[7] N. A. Tatton and S. J. Kish. In situ detection of apoptotic nuclei in the substantia aigra compacta of 1-methyl-4-phenyl-1,2,3,6-tetrahydropyridine-treated mice using terminal deoxynucleotidyl transferase labeling and acridine orange staining [J].Neuroscience Vol.77, No.4,pp.1037-1048, 1997.

[8] D J Drucker, J Philippe, S Mojsov et al. Glucagon-like peptide I stimulates insulin gene expression and increases cyclic AMP levels in a rat islet cell line. Proc Natl Acad Sci U S A. 1987 May; 84(10): 3434-3438.

[9] Mojsov S, Weir GC, Habener JF. Insulinotropin: glucagon-like peptide I (7-37) co-encoded in the glucagon gene is a potent stimulator of insulin release in the perfused rat pancreas. J Clin Invest. 1987 Feb;79(2):616-9.

[10] Li Y, Perry T, Kindy M.S, et al. (2009). GLP-1 receptor stimulation preserves primary cortical and dopaminergic neurons in cellular and rodent models of stroke and Parkinsonism. Proc. Natl. Acad. Sci. USA.106, 1285-1290.

[11] Toshiharu Nagatsu, Akria Nakashima, et al. Human tyrosine hydroxylase in Parkinson's disease and in related disorders. Journal of Neural Transmission, 2019, 126:397-409.

[12] Lee TI, Yang CS, Fang KM, Tzeng SF (2009) Role of ciliary neurotrophic factor in microglial phagocytosis. Neurochem Res 34:109-117.

[13] Liuzzi GM, Latronico T, Rossano R, Viggiani S, Fasano A,Riccio P (2007) .Inhibitory effect of polyunsaturated fatty acids on MMP-9 release from microglia-implications for complementary multiple sclerosis treatment. Neurochem Res 32:2184-2193.

[14] Pasquini LA, Calatayud CA, Bertone Un~a AL, Millet V, Pasquini JM, Soto EF (2007) The neurotoxic effect of cuprizone on oligodendrocytes depends on the presence of pro-inflammatory cytokines secreted by microglia. Neurochem Res 32:279-292.

[15] Hirasawa T, Ohsawa K, Imai Y et al (2005) Visualization of microglia in living tissues using Iba1-EGFP transgenic mice.J Neurosci Res 81:357-362.

[16] Kalm M, Lannering B, Bjo"rk-Eriksson T, Blomgren K (2009). Irradiation-induced loss of microglia in the young brain. J Neuroimmunol 206:70-75.

[17] Li ZH, Lu J, Tay SS, Wu YJ, Strong MJ, He BP (2006) Mice with targeted disruption of neurofilament light subunit display formation of protein aggregation in motoneurons and downregulation of complement receptor type 3 alpha subunit in microglia in the spinal cord at their earlier age: a possible feature in preclinical development of neurodegenerative diseases. Brain Res 1113:200-209.

[18] Khodanovich M Y, Sorokina I V, GIazacheva V Y, et a1. Histological validation of fast macromolecular proton fraction mapping as a quantitative myelin imaging method in the cuprizone demyelination mode[J]. Sci Rep, 2017, 7:46686.

[19] Wang C, Sun C, Hu Z, et al. Improved neural regeneration with olfactory ensheathing cell inoculated PLGA scaffolds in spinal cord injury adult rats [J].N eurosignals,20 17,25(1): 1-14.

[20] KALIA L V, KALIA S K. Alpha-Synuclein and Lewy pathology in Parkinson's disease[J]. Curr Opin Neurol, 2015, 28 (4): 375-381.

[21] Julien JP, Mushynski WE. Neurofilaments in health and disease[J]. Prog Nucleic Acid Res Mol Biol,1998,61:1-23.

[22] Middeldorp J, Hol E. GFAP in health and disease[J]. Prog Neurobiol, 2011, 93:421-443.

[23] Yokoyama H, Uchida H, Kuroiwa H, et al. Role of glial cells in neurotoxin induced animal models of Parkinson's disease[J]. Neurol Sci, 2011, 32(1):1-7.

[24] Kowianski P, Lietzau G, Czuba E, Waskow M, Steliga A, Morys J. BDNF: A Key Factor with Multipotent Impact on Brain Signaling and Synaptic Plasticity. Cell Mol Neurobiol. 2018 Apr;38(3):579-593.

**Claims**

1. A method for preventing and treating Parkinson's disease, comprising: administering a therapeutically effective amount of one or more compounds to a subject with Parkinson's disease, the one or more compounds being selected from the group consisting of: a component of a plasminogen activation pathway, a compound capable of directly activating plasminogen or indirectly activating plasminogen by activating an upstream component of the plasminogen activation pathway, a compound mimicking the activity of plasminogen or plasmin, a compound capable of up-regulating the expression of plasminogen or a plasminogen activator, a plasminogen analog, a plasmin analog, a tPA or uPA analog and an antagonist of a fibrinolysis inhibitor.

2. The method according to claim 1, wherein the component of the plasminogen activation pathway is selected from the group consisting of plasminogen, recombinant human plasmin, Lys-plasminogen, Glu-plasminogen, plasmin, plasminogen and plasmin variants and analogs containing one or more kringle domains and protease domains of plasminogen and plasmin, mini-plasminogen, mini-plasmin, micro-plasminogen, micro-plasmin, delta-plasminogen, delta-plasmin, a plasminogen activator, tPA and uPA.

3. The method according to claim 1, wherein the antagonist of the fibrinolysis inhibitor is an inhibitor of PAI-1, a complement C1 inhibitor, $\alpha2$ antiplasmin or $\alpha2$ macroglobulin, such as, an antibody.

4. The method according to any one of claims 1 to 3, wherein the compound has one or more of the following effects on a subject with Parkinson's disease: promoting the recovery of a memory function, improving the cognitive ability, promoting the expression of DTA in the substantia nigra, promoting the recovery of the Nissl body of striatum, promoting the expression of GLP-1R in the substantia nigra, increasing the number of TH-positive cells in the substantia nigra, promoting myelin sheath repair of striatum, promoting the degradation of $\alpha$-synuclein in brain tissues, promoting the NF expression of myelin, promoting the axonal damage repair, reducing the GFAP expression of myelin, reducing the neuron damage of myelin, promoting the cleavage of Pro-BDNF in brain tissues into BDNF, and relieving the depression or anxiety symptoms.

5. The method according to any one of claims 1 to 4, wherein the compound is plasminogen.

6. The method according to any one of claims 1 to 5, wherein the plasminogen is human full-length plasminogen or a conservatively substituted variant thereof.

7. The method according to any one of claims 1 to 5, wherein the plasminogen has at least 75%, 80%, 85%, 90%, 95%, 96%, 97%, 98% or 99% sequence identity to sequence 2 and still has a lysine binding activity or proteolytic activity of the plasminogen.

8. The method according to any one of claims 1 to 5, wherein the plasminogen contains a protein that has an amino acid sequence having at least 80%, 90%, 95%, 96%, 97%, 98% or 99% amino acid sequence identity to sequence 14 and still has the proteolytic activity of the plasminogen.

9. The method according to any one of claims 1 to 5, wherein the plasminogen is selected from the group consisting of Glu-plasminogen, Lys-plasminogen, small plasminogen, microplasminogen, delta-plasminogen or their variants that retain the proteolytic activity of the plasminogen.

10. The method according to any one of claims 1 to 5, wherein the plasminogen contains an amino acid sequence shown in sequence 2, 6, 8, 10, 12 or a conservatively substituted variant containing an amino acid sequence shown in sequence 2, 6, 8, 10, 12.

11. The method according to any one of claims 1 to 10, wherein the compound is used in combination with one or more other therapeutic methods or drugs.

12. The method according to claim 11, wherein the other therapeutic methods comprise a cell therapy (comprising a stem cell therapy) and a physical therapy.

13. The method according to claim 11, wherein the other drugs are other drugs for the treatment of Parkinson's disease.

14. The method according to any one of claims 1 to 13, wherein the compound is administered by means of nasal inhalation, aerosol inhalation, nasal drops, eye drops, ear drops, an intravenous method, an intraperitoneal method, a subcutaneous method, an intracranial method, an intrathecal method, an intraarterial method (e.g., via the carotid artery) or an intramuscular method.

Figure 1

Figure 2

Figure 3

Figure 4

Figure 5

Figure 6

Figure 7

Figure 8

Figure 9

Figure 10

Figure 11

Figure 12

Blank control group

Solvent group

PLG

Figure 13

Figure 14

Figure 15

Figure 16

Figure 17

Figure 18

Figure 19

Figure 20

Figure 21

Figure 22

Figure 23

Figure 24

Figure 25

Figure 26

Figure 27

## INTERNATIONAL SEARCH REPORT

International application No.

**PCT/CN2021/082715**

| A. CLASSIFICATION OF SUBJECT MATTER |
| --- |
| A61K 38/48(2006.01)i; A61P 25/16(2006.01)i; C07K 14/435(2006.01)i |

According to International Patent Classification (IPC) or to both national classification and IPC

| B. FIELDS SEARCHED |
| --- |

Minimum documentation searched (classification system followed by classification symbols)

A61K; C07K

Documentation searched other than minimum documentation to the extent that such documents are included in the fields searched

Electronic data base consulted during the international search (name of data base and, where practicable, search terms used)

VEN, CNABS, PubMed, ISI Web of Science, CNTXT, WOTXT, EPTXT, USTXT, CNKI, 万方, WANFANG, 百度学术, BAIDU XUESHU, 读秀, DUXIU, STN, GenBank, 中国专利生物序列检索系统, CHINESE PATENT BIOLOGICAL SEQUENCE RETRIEVAL SYSTEM: parkinson, plasminogen, SEQ ID NO:2, 4, 6, 8, 纤溶酶原, 帕金森

| C. DOCUMENTS CONSIDERED TO BE RELEVANT |
| --- |

| Category* | Citation of document, with indication, where appropriate, of the relevant passages | Relevant to claim No. |
| --- | --- | --- |
| X | CN 109125715 A (SHENZHEN RUIJIAN LIFE SCIENCE RESEARCH INSTITUTE CO., LTD.) 04 January 2019 (2019-01-04) claims 1-17, and sequence listing | 1-14 |
| X | PAN, H. et al. "Role of Plasminogen Activator Inhibitor-1 in the Diagnosis and Prognosis of Patients with Parkinson's Disease" *Experimental and Therapeutic Medicine*, Vol. 15, 31 December 2018 (2018-12-31), the abstract | 1-14 |
| X | JEON, H. et al. "Plasminogen Activator Inhibitor Type 1 Regulates Microglial Motility and Phagocytic Activity" *Journal of Neuroinflammation*, Vol. 9, 31 December 2012 (2012-12-31), the abstract | 1-14 |
| A | WO 2011147999 A1 (FARMALIDER, S.A.) 01 December 2011 (2011-12-01) entire document | 1-14 |
| A | WO 0124784 A2 (FUJISAWA PHARMACEUTICAL CO., LTD.) 12 April 2001 (2001-04-12) entire document | 1-14 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **03 June 2021** | **23 June 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/082715**

**C.     DOCUMENTS CONSIDERED TO BE RELEVANT**

| Category* | Citation of document, with indication, where appropriate, of the relevant passages | Relevant to claim No. |
|---|---|---|
| A | 陈武 等 (CHEN, Wu et al.). "纤溶酶原受体与相关疾病的分子机制 (Molecular Mechanisms of Plasminogen Receptors and Associated Diseases)" 生物化学与生物物理进展 (*Progress in Biochemistry and Biophysics*), Vol. 42, No. 12, 31 December 2015 (2015-12-31), 1094-1102 | 1-14 |
| A | 曾克军 等 (ZENG, Kejun et al.). "α-synuclein和帕金森病的关系研究进展 (Non-official translation: Advances in the Relationship between α-synuclein and Parkinson's Disease)" 国际神经病学神经外科学杂志 (*Journal of International Neurology and Neurosurgery*), Vol. 39, No. 5, 31 December 2012 (2012-12-31), 468-471 | 1-14 |

Form PCT/ISA/210 (second sheet) (January 2015)

**INTERNATIONAL SEARCH REPORT**

| International application No. |
|---|
| **PCT/CN2021/082715** |

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2021/082715**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **1-14**
   because they relate to subject matter not required to be searched by this Authority, namely:

   [1]  Claims 1-14 relate to methods for the prevention and treatment of Parkinson's disease and are methods of treating the disease as defined in PCT Rule 39.1(iv); therefore a search is made on the basis of a hypothetical technical solution for pharmaceutical use of components of the fibrinogen activation pathway.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
**Information on patent family members**

International application No.

**PCT/CN2021/082715**

| Patent document<br>cited in search report | | | Publication date<br>(day/month/year) | Patent family member(s) | | | Publication date<br>(day/month/year) |
|---|---|---|---|---|---|---|---|
| CN | 109125715 | A | 04 January 2019 | None | | | |
| WO | 2011147999 | A1 | 01 December 2011 | EP | 2578212 | A4 | 06 November 2013 |
| | | | | EP | 2578212 | A1 | 10 April 2013 |
| | | | | US | 9000047 | B2 | 07 April 2015 |
| | | | | US | 2013065966 | A1 | 14 March 2013 |
| | | | | EP | 2578212 | B1 | 06 July 2016 |
| | | | | PL | 2578212 | T3 | 31 January 2017 |
| WO | 0124784 | A2 | 12 April 2001 | WO | 0124784 | A3 | 10 May 2002 |
| | | | | JP | 2003510351 | A | 18 March 2003 |
| | | | | EP | 1223969 | A2 | 24 July 2002 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102154253 A
- WO 9704801 A

- US 3773919 A

### Non-patent literature cited in the description

- **NY, A. ; LEONARDSSON, G. ; HAGGLUND, A.C ; HAGGLOF, P. ; PLOPLIS, V.A. ; CARMELIET, P. ; NY, T.** Ovulation inplasminogen-deficient mice. *Endocrinology,* 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.,* November 1984, vol. 74 (5), 1625-33
- **GRAVANIS I ; TSIRKA SE.** Tissue-type plasminogen activator as a therapeutic target in stroke. *Expert Opinion on Therapeutic Targets.,* February 2008, vol. 12 (2), 159-70
- **GEIGER M ; HUBER K ; WOJTA J ; STINGL L ; ESPANA F ; GRIFFIN JH ; BINDER BR.** Complex formation between urokinase and plasma protein C inhibitor in vitro and in vivo. *Blood,* August 1989, vol. 74 (2), 722-8
- **AISINA R B ; MUKHAMETOVA L I.** Structure and function of plasminogen/plasmin system. *Russian Journal of Bioorganic Chemistry,* 2014, vol. 40 (6), 590-605
- **BARANY et al.** Special Methods in Peptide Synthesis. *Solid-Phase Peptide Synthesis; The Peptides: Analysis, Synthesis, Biology,* vol. 2, 3-284
- **MERRIFIELD et al.** Solid Phase Peptide Synthesis. I. The Synthesis of a Tetrapeptide. *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277

- **LANGER.** *Chem. Tech.,* 1982, vol. 12, 98-105
- **SIDMAN et al.** *Biopolymers,* 1983, vol. 22, 547
- **KENNETH C ROBBINS ; LOUIS SUMMARIA ; DAVID ELWYN et al.** Further Studies on the Purification and Characterization of Human Plasminogen and Plasmin. *Journal of Biological Chemistry,* 1965, vol. 240 (1), 541-550
- **SUMMARIA L ; SPITZ F ; ARZADON L et al.** Isolation and characterization of the affinity chromatography forms of human Glu- and Lys-plasminogens and plasmins. *J Biol Chem.,* 25 June 1976, vol. 251 (12), 3693-9
- **HAGAN JJ ; ABLONDI FB ; DE RENZO EC.** Purification and biochemical properties of human plasminogen. *J Biol Chem.,* April 1960, vol. 235, 1005-10
- **M. SEDELIS et al.** Behavioral phenotyping of the MPTP mouse model of Parkinson's Disease. *Beha ioural Brain Research,* 2001, vol. 125, 109-122
- **MEREDITH G E ; TOTTERDELL S ; POTASHKIN J A.** Modeling PD pathogenesis in mice advantages of a chronic MPTP protocol. *Parkinsonism Relat Disord,* 2008, vol. 14, S112-S115
- **KEN IKEDA ; JUNYA EBINA ; KIYOKAZU KAWABE ; YASUO IWASAKI.** Dopamine Transporter Imaging in Parkinson Disease:Progressive Changes and Therapeutic Modification after Anti-parkinsonian Medications. *Internal Medicine,* 2019, vol. 58, 1665-1672
- **HEBERT AE, DASH PK.** Nonredundant roles for hippocampal and entorhinal cortical plasticity in spatial memory storage. *Pharmacology Biochemistry and Behavior,* 2004, vol. 79 (1), 143-153
- **WON-SEOK CHOI et al.** Conditional deletion of Ndufs4 in dopaminergic neurons promotes Parkinson's disease-like nonmotor symptoms without loss of dopamine neurons. *Scientific Reports*
- **VERNICE JACKSON-LEWIS1 ; SERGE PRZEDBORSKI.** Protocol for the MPTP mouse model of Parkinson's Disease. *Nature protocols,* 2007, vol. 2 (1), 141

- **N. A. TATTON ; S. J. KISH.** In situ detection of apoptotic nuclei in the substantia aigra compacta of 1-methyl-4-phenyl-1,2,3,6-tetrahydropyridine-treated mice using terminal deoxynucleotidyl transferase labeling and acridine orange staining. *Neuroscience,* 1997, vol. 77 (4), 1037-1048
- **D J DRUCKER ; J PHILIPPE ; S MOJSOV et al.** Glucagon-like peptide I stimulates insulin gene expression and increases cyclic AMP levels in a rat islet cell line. *Proc Natl Acad Sci U S A.,* May 1987, vol. 84 (10), 3434-3438
- **MOJSOV S ; WEIR GC ; HABENER JF.** Insulinotropin: glucagon-like peptide I (7-37) co-encoded in the glucagon gene is a potent stimulator of insulin release in the perfused rat pancreas. *J Clin Invest.,* February 1987, vol. 79 (2), 616-9
- **LI Y ; PERRY T ; KINDY M.S et al.** GLP-1 receptor stimulation preserves primary cortical and dopaminergic neurons in cellular and rodent models of stroke and Parkinsonism. *Proc. Natl. Acad. Sci. USA.,* 2009, vol. 106, 1285-1290
- **TOSHIHARU NAGATSU ; AKRIA NAKASHIMA et al.** Human tyrosine hydroxylase in Parkinson's disease and in related disorders. *Journal of Neural Transmission,* 2019, vol. 126, 397-409
- **LEE TI ; YANG CS ; FANG KM ; TZENG SF.** Role of ciliary neurotrophic factor in microglial phagocytosis. *Neurochem Res,* 2009, vol. 34, 109-117
- **LIUZZI GM ; LATRONICO T ; ROSSANO R ; VIGGIANI S ; FASANO A ; RICCIO P.** Inhibitory effect of polyunsaturated fatty acids on MMP-9 release from microglia-implications for complementary multiple sclerosis treatment. *Neurochem Res,* 2007, vol. 32, 2184-2193
- **PASQUINI LA ; CALATAYUD CA ; BERTONE UN~A AL ; MILLET V ; PASQUINI JM ; SOTO EF.** The neurotoxic effect of cuprizone on oligodendrocytes depends on the presence of pro-inflammatory cytokines secreted by microglia. *Neurochem Res,* 2007, vol. 32, 279-292
- **HIRASAWA T ; OHSAWA K ; IMAI Y et al.** Visualization of microglia in living tissues using Iba1-EGFP transgenic mice. *J Neurosci Res,* 2005, vol. 81, 357-362
- **KALM M ; LANNERING B ; BJO"RK-ERIKSSON T ; BLOMGREN K.** Irradiation-induced loss of microglia in the young brain. *J Neuroimmunol,* 2009, vol. 206, 70-75
- **LI ZH ; LU J ; TAY SS ; WU YJ ; STRONG MJ ; HE BP.** Mice with targeted disruption of neurofilament light subunit display formation of protein aggregation in motoneurons and downregulation of complement receptor type 3 alpha subunit in microglia in the spinal cord at their earlier age: a possible feature in preclinical development of neurodegenerative diseases. *Brain Res,* 2006, vol. 1113, 200-209
- **KHODANOVICH M Y ; SOROKINA I V ; GIAZACHEVA V Y.** Histological validation of fast macromolecular proton fraction mapping as a quantitative myelin imaging method in the cuprizone demyelination mode. *Sci Rep,* 2017, vol. 7, 46686
- **WANG C ; SUN C ; HU Z et al.** Improved neural regeneration with olfactory ensheathing cell inoculated PLGA scaffolds in spinal cord injury adult rats. *N eurosignals,* 2017, vol. 25 (1), 1-14
- **KALIA L V ; KALIA S K.** Alpha-Synuclein and Lewy pathology in Parkinson's disease. *Curr Opin Neurol,* 2015, vol. 28 (4), 375-381
- **JULIEN JP ; MUSHYNSKI WE.** Neurofilaments in health and disease. *Prog Nucleic Acid Res Mol Biol,* 1998, vol. 61, 1-23
- **MIDDELDORP J.** Hol E. GFAP in health and disease. *Prog Neurobiol,* 2011, vol. 93, 421-443
- **YOKOYAMA H ; UCHIDA H ; KUROIWA H et al.** Role of glial cells in neurotoxin induced animal models of Parkinson's disease. *Neurol Sci,* 2011, vol. 32 (1), 1-7
- **KOWIANSKI P, LIETZAU G, CZUBA E, WASKOW M, STELIGA A, MORYS J. BDNF.** A Key Factor with Multipotent Impact on Brain Signaling and Synaptic Plasticity. *Cell Mol Neurobiol.,* April 2018, vol. 38 (3), 579-593